# EUROPEAN PATENT SPECIFICATION

(11) **EP 3 146 340 B1**
(45) Date of publication and mention of the grant of the patent: **22.04.2020**
(21) Application number: 15727217.0
(22) Date of filing: 21.05.2015
(51) Int. Cl.: G01N 33/569

(54) **METHODS OF PREPARING MATERIALS WITH AMMONIA OXIDIZING BACTERIA AND TESTING MATERIALS FOR AMMONIA OXIDIZING BACTERIA**
VERFAHREN ZUR HERSTELLUNG VON MATERIALIEN MIT AMMONIAKOXIDIERENDEN BAKTERIEN UND PRÜFUNG VON MATERIALIEN FÜR AMMONIAKOXIDIERENDE BAKTERIEN
PROCÉDÉS DE PRÉPARATION DE MATÉRIAUX AU MOYEN DE BACTÉRIES OXYDANT L'AMMONIAC ET DE TEST DE MATÉRIAUX EN CE QUI CONCERNE DES BACTÉRIES OXYDANT L'AMMONIAC

(30) Priority: 22.05.2014 US 201462002028 P
(43) Date of publication of application: 29.03.2017
(73) Proprietor: Aobiome LLC, Cambridge, MA 02142 (US)
(72) Inventor: WHITLOCK, David, R., Cambridge, MA 02140 (US); HEYWOOD, James, Newton, MA 02461 (US); JAMAS, Spiros, Cambridge, MA 02138 (US); WEISS, Larry, San Francisco, CA 94132 (US)
(74) Representative: Titmus, Craig Edward
(86) International application number: PCT/US2015/032017
(87) International publication number: WO 2015/179669

(56) References cited:
- WO-A2-2015/160911
- US-A1- 2004 014 188
- US-A1- 2007 148 136
- US-A1- 2008 213 226
- JUNTAEK LIM ET AL: "Primer and probe sets for group-specific quantification of the generaNitrosomonas andNitrosospira using real-time PCR", BIOTECHNOLOGY AND BIOENGINEERING, vol. 99, no. 6, 1 January 2008 (2008-01-01), pages 1374-1383, XP055205690, ISSN: 0006-3592, DOI: 10.1002/bit.21715
- PUNTIPAR SONTHIPHAND ET AL: "Evaluating Primers for Profiling Anaerobic Ammonia Oxidizing Bacteria within Freshwater Environments", PLOS ONE, vol. 8, no. 3, 7 March 2013 (2013-03-07), page e57242, XP055205585, DOI: 10.1371/journal.pone.0057242
- A. HERMANSSON ET AL: "Quantification of Ammonia-Oxidizing Bacteria in Arable Soil by Real-Time PCR", APPLIED AND ENVIRONMENTAL MICROBIOLOGY, vol. 67, no. 2, 1 February 2001 (2001-02-01), pages 972-976, XP055205638, ISSN: 0099-2240, DOI: 10.1128/AEM.67.2.972-976.2001
- S. L. DOLLHOPF ET AL: "Quantification of Ammonia-Oxidizing Bacteria and Factors Controlling Nitrification in Salt Marsh Sediments", APPLIED AND ENVIRONMENTAL MICROBIOLOGY, vol. 71, no. 1, 1 January 2005 (2005-01-01), pages 240-246, XP055205637, ISSN: 0099-2240, DOI: 10.1128/AEM.71.1.240-246.2005

## Description

### Background

Beneficial bacteria can be used to suppress the growth of pathogenic bacteria. Bacteria and other microorganisms are ubiquitous in the environment. The discovery of pathogenic bacteria and the germ theory of disease have had a tremendous effect on health and disease states. Bacteria are a normal part of the environment of all living things. In the gut, these bacteria are not pathogenic under normal conditions, and in fact improve health by rendering the normal intestinal contents less hospitable for disease causing organisms. Disease prevention is accomplished in a number of ways: nutrients are consumed, leaving less for pathogens; conditions are produced, such as pil and oxygen tension, which arc not hospitable for pathogens; compounds are produced that are toxic to pathogens; pathogens are consumed as food by these microorganisms; less physical space remains available for pathogens; and specific hinding sites are occupied leaving fewer binding sites available for pathogens. The presence of these desirable bacteria is seen as useful in preventing disease states.

US 2004/014188 relates to compositions including ammonia oxidizing bacteria to increase production of nitric oxide and nitric oxide precursors and methods of using same; US 2008/213226 relates to compositions including ammonia oxidizing bacteria and methods of using same; US 2007/148136 relates to methods of using ammonia oxidizing bacteria. Juntaek Lim *et al* (2008) relates to primer and probe sets for group-specific quantification of the genera *Nitrosomonas* and *Nitrosospira* using real-time PCR. Puntipar Sonthiphand *et al.* (2013) refers to evaluating primers for profiling anaerobic ammonia oxidizing bacteria within freshwater environments. Hermansson *et al.* (2001) relates to the quantification of ammonia oxidizing bacteria in arable soil by real-time PCR, and Dollhopf *et al.* (2005) relates to the quantification of ammonia oxidizing bacteria and factors controlling nitrification in salt marsh sediments.

There is a need in the art for improved beneficial bacteria that can suppress the growth of non-autotrophic bacteria, *e.g*., pathogenic bacteria.

### Summary

Based on the disclosure that is contained herein, the present invention provides a method of evaluating a preparation for the level of ammonia oxidizing bacteria, comprising:
(a) measuring an amount or a concentration of ammonia oxidizing bacteria in the preparation to provide a value;
(b) comparing the value to a range of values corresponding to a pre-determined range of amounts or concentrations of ammonia oxidizing bacteria; and
(c) determining if the value is a value in the range of values corresponding to the pre-determined range of amounts or concentrations of ammonia oxidizing bacteria, wherein:
   (i) if the value is in the range of values corresponding to the pre-determined range of amounts or concentrations of ammonia oxidizing bacteria, classifying the product as accepted; or
   (ii) if the value is outside the range of values corresponding to the pre-determined range of amounts or concentrations of ammonia oxidizing bacteria, classifying the product as not accepted;
wherein the preparation is a cosmetic product, a therapeutic product or a consumer product, or is in the process of being prepared or manufactured as a cosmetic product, a therapeutic product or a consumer product.

The present invention and some preferred embodiments thereof are set out in the appended claims.

This disclosure provides, *inter alia,* a method of evaluating a preparation for the presence, ***e.g***., level, of ammonia oxidizing bacteria, *e*.*g*., a cosmetic product or a therapeutic product, comprising measuring an amount of a concentration of ammonia oxidizing bacteria in the product to provide a value.

Ammonia oxidizing bacteria are ubiquitous Gram-negative obligate chemolithoautotropic bacteria with a unique capacity to generate energy exclusively from the conversion of ammonia to nitrite.

Ammonia oxidizing bacteria catalyze the following reactions.

At a neutral pH, ammonia generated from ammonium around neutral pH conditions is the substrate of the initial reaction. The conversion of ammonia to nitrite takes place in two steps catalyzed respectively by ammonia monooxygenase (Amo) and hydroxylamine oxidoreductase (Hao), as follows:

NH₃ + 2H⁺ + 2e⁻ + O₂ → NH₂OH + H₂O (A)

NH₂OH + H₂O → NO₂⁻ + 4e⁻ + 5H⁺ (B)

In some instances, reaction B is reported as follows, to indicate nitrous acid (HNO₂) formation at low pH:

NH₂OH + H₂O → HNO₂ + 4e⁻ + 4H⁺

NH₄⁺ and NH₃ may be used interchangeably throughout the disclosure.

### Detailed Description

The present disclosure provides for methods of preparing materials with ammonia oxidizing bacteria. The present disclosure also provides for methods of testing materials for ammonia oxidizing bacteria. Preparations, compositions, and formulations including, *e*.*g*., natural products and fortified natural products, comprising ammonia oxidizing bacteria are contemplated.

Ammonia-oxidizing bacteria (AOB) of the genus *Nitrosomonas* are Gram-negative obligate autotrophic bacteria with a unique capacity to generate nitrite and nitric oxide exclusively from ammonia as an energy source. They are widely present both in soil and water environments and are essential components of environmental nitrification processes. Due to the roles of nitrite and nitric oxide on human skin as important components of several physiological functions, such as vasodilation, skin inflammation and wound healing, these bacteria may have beneficial properties for both healthy and immunopathological skin conditions. These bacteria may be safe for use in humans because they are slow-growing, cannot grow on organic carbon sources, may be sensitive to soaps and antibiotics, and have never been associated with any disease or infection in animals or humans.

### 1. Definitions

An ammonia oxidizing bacterium refers to a bacterium capable of oxidizing ammonia or ammonium to nitrite. This may be accomplished at a rate. The rate, *e.g.*, a pre-determined rate, may refer to the conversion of ammonium ions (NH₄⁺) (*e.g.*, at about 200 mM) to nitrite (NO₂⁻) at a rate of at least 50, 75, 125, or 150 micromoles NO₂⁻ per minute, *e.g.*, about 100-150, 75-175, 75-125, 100-125, 125-150, or 125-175 micromoles/minute, *e.g.*, about 125 micromoles NO₂⁻ per minute. Examples of ammonia oxidizing bacteria include *Nitrosomonas eutropha* strains, *e.g.*, D23 and C91, and other bacteria in the genera *Nitrosomonas*, *Nitrosococcus*, *Nitrosospira*, *Nitrosocystis, Nitrosolobus,* and *Nitrosovibrio.* D23 *Nitrosomonas eutropha* strain refers to the strain, designated AOB D23-100, deposited with the American Tissue Culture Collection (ATCC) (10801 University Blvd., Manassas, VA, USA) on April 8, 2014 having accession number PTA-121157.

In certain instances, the *N. eutropha* is a strain described in PCT Application No. PCT/US2015/025909, filed April 15, 2015.

As used herein, "axenic" refers to a composition comprising an organism that is substantially free of other organisms. For example, an axenic culture of ammonia oxidizing bacteria is a culture that is substantially free of organisms other than ammonia oxidizing bacteria; "substantially free" denotes undetectable by a method used to detect other organisms, *e*.*g*., plating the culture and examining colony morphology, or PCR for a conserved gene such as 16S RNA. An axenic composition may comprise elements that are not organisms, *e*.*g*., it may comprise nutrients or excipients. Any preparation, composition, or formulation of ammonia oxidizing bacteria discussed herein may comprise, consist essentially of, or consist of optionally axenic ammonia oxidizing bacteria.

As used herein, an "autotroph", *e.g.,* an autotrophic bacterium, is any organism capable of self-nourishment by using inorganic materials as a source of nutrients and using photosynthesis or chemosynthesis as a source of energy. Autotrophic bacteria may synthesize organic compounds from carbon dioxide and ATP derived from other sources, oxidation of ammonia to nitrite, oxidation of hydrogen sulfide, and oxidation of Fe2⁺ to Fe3⁺ Autotrophic bacteria of the present disclosure are incapable of causing infection.

To "culture" refers to a process of placing an amount of a desired bacterium under conditions that promote its growth, *i.e.*, promoting cell division. The conditions can involve a specified culture medium, a set temperature range, and/or an agitation rate. Bacteria can be cultured in a liquid culture or on plates, *e.g.,* agar plates.

"Activation," as used herein, is used relative to autotrophic bacteria, *e*.*g*., ammonia oxidizing bacteria. Activation refers to any action that may place the ammonia oxidizing bacteria in a potentially more active state, *e.g.,* a growth state. Activation may relate to stimulation of autotrophic bacteria, *e*.*g*., ammonia oxidizing bacteria, to assist in some way in the conversion of at least one of ammonia, ammonium ions, and urea into nitrite, nitric oxide, or nitric oxide precursors. Activation may relate to helping establish a bacterial colony, *e.g.,* to allow for the autotrophic bacteria, *e*.*g*., ammonia oxidizing bacteria, to compete with other existing bacteria. Activation may relate to providing an environment that may favor sustainability and/or growth of autotrophic bacteria, *e*.*g*., ammonia oxidizing bacteria. Activation may relate to accelerating availability of the autotrophic bacteria, *e*.*g*., ammonia oxidizing bacteria to an environment or a surface. "Activation" may provide for ammonia oxidizing bacteria to be in an "activated" or "growth state." "Activation" may take place with the use of an activator. The ammonia oxidizing bacteria may come into contact with the activator to provid an ammonia oxidizing bacteria in an "activated" or "growth" state. This may occur within or outside of a container, delivery device, or delivery system, *e*.*g*., within a first chamber, a second chamber, a mixing chamber, a third or additional chamber, or combinations thereof. The activator may be at least one of ammonia, ammonium ions, or urea. The activator may be an ammonium salt, *e*.*g*., ammonium chloride or ammonium sulfate. The concentration of the activator, *e.g.,* ammonium salt, *e.g.,* ammonium chloride or ammonium sulfate may be in a range of about 10 micromolar to about 100 millimolar. In certain aspects the concentration of the activator, *e.g.,* ammonium salt, *e.g.,* ammonium chloride or ammonium sulfate may be in a range of about 0.5 mM to about 50 mM. The activator may be in a solution, suspension, a powder, *e.g.*, crystalline form, a media, a buffer, or disposed in or provide as a suitable carrier for maintaining the activator. The ammonia oxidizing bacteria may be in any suitable form for maintaining the AOB in a desired state, *e.g.,* a storage state, *e.g.,* an aqueous suspension, gel, or powder form. The at least one of ammonia, ammonium ions, or urea may be in a medium or a buffer to promote growth of ammonia oxidizing bacteria, *e.g.,* an AOB media or a growth media. A time-release, or controlled release urea may be used as an activator.

"Actuation," as used herein, means that some action is being taken, *e.g.,* a process is being started or something is being put into motion. Actuation may refer to the breaking of a barrier of a container, or the initiation of movement of one or more contents of a container, *e.g.,* delivery of one or more contents of the container to outside of the container, *e.g.,* to a surface or an environment.

A "barrier," as used herein, may mean any structure or configuration that may serve to obstruct passage or to maintain separation, *e.g.,* between a first chamber and a second chamber of a container. The barrier may be in the form of a valve, *e.g.,* a check valve, filtering material, film, wax, lipid, polymer, or controlled release material, *e*.*g*., slow release material. The barrier may be a material that upon actuation of a container, it may allow passage of contents from a first chamber into a second chamber, passage of contents from a second chamber into a first chamber, or both. The barrier may be disrupted upon actuation, *e.g.,* through piercing, puncturing, stabbing, perforating, penetrating, splitting, opening or tearing the barrier. The barrier may be in a form of a valve, *e.g.,* a check valve, a flexible or inflexible material that may not degrade upon contact with one or more contents of the container, or a flexible or inflexible material that may degrade upon contact with one or more contents of the container, a filter material. The barrier may be made of any material suitable for its purpose, *e.g.,* a material that may serve to obstruct passage or to maintain separation, *e.g.,* a polymeric material or metal material.

In some instances, the states most relevant to the present disclosure are the state of growth, *e*.*g*., maximal growth, characterized by a pH of at least about 7.6, ammonia, trace minerals, oxygen and carbon dioxide. Another state may be characterized by a pH of about 7.4 or less and characterized by an absence of carbon dioxide. Under low carbon dioxide conditions, ammonia oxidizing bacteria, *e.g., Nitrosomonas,* continues to oxidize ammonia into nitrite and generates ATP, but lacking carbon dioxide, *e*.*g*., lacking sufficient carbon dioxide, to fix and generate protein, it instead generates polyphosphate, which it uses as an energy storage medium. This may allow the ammonia oxidizing bacteria to remain in a "storage state" for a period of time, *e.g.,* a pre-determined period of time, for example, at least 1, 2, 3, 4, 5, 6, 7, days, 1, 2, 3, 4 weeks, 1, 2, 3, 4, 5, 6, 7, 8, 9, 10, 11, 12 months, 1, 2, 3, 4, or 5 years. In some instances, the ammonia oxidizing bacteria may remain in a storage state for at least about 6 months to about 1 year.

As used herein, "growth state" refers to autotrophic bacteria, *e*.*g*., ammonia oxidizing bacteria, in a state or in an environment, *e.g.,* a media, *e.g.,* a culture media, *e.g.,* a growth media, that may have a pH of at least about 7.6. Levels of at least one of ammonia, ammonium ions, and urea may be between about 1 micromolar and 1000 millimolar. Levels of trace materials are between about 0.01 micromolar iron and 200 micromolar iron. Levels of oxygen are between about 5% and 100% oxygen saturation (*e.g*., of media). Levels of carbon dioxide are between about 20 ppm and 10% saturation (*e.g*., of media). In certain aspects, levels of at least one of ammonia, ammonium ions, and urea may be between about 10 micromolar and 100 millimolar. Levels of trace materials are between about 0.1 micromolar iron and 20 micromolar iron. Levels of oxygen are between about 5% and 100% oxygen saturation. Levels of carbon dioxide are between about 200 ppm and 5% saturation (*e.g*., of media).

As used herein, "polyphosphate loading state" refers to autotrophic bacteria, *e*.*g*., ammonia oxidizing bacteria, in a state or in an environment, *e.g.,* a media, *e.g.,* a culture media, *e.g.,* a growth media, that may have a pH of about 7.4, or less. Levels of at least one of ammonia, ammonium ions, and urea are between about 1 micromolar and 2000 millimolar. Levels of trace materials are between 0.01 micromolar iron and 200 micromolar iron. Levels of oxygen are between about 0% and 100% O₂ saturation (*e*.*g*., of media). Levels of carbon dioxide are between/less than about zero and 400 ppm, and phosphate levels greater than about 1 micromolar. In certain aspects, levels of at least one of ammonia, ammonium ions, and urea are between about 10 micromolar and 200 millimolar. Levels of trace materials are between 0.1 micromolar iron and 20 micromolar iron. Levels of oxygen are between about 5% and 100% O₂ saturation. Levels of carbon dioxide are between/less than about zero and 200 ppm, and phosphate levels greater than about 10 micromolar.

The polyphosphate loading state may be induced for a period of time, *e*.*g*., a pre-determined period of time. The pre-determined period of time may be the time period that allows sufficient polyphosphate accumulation in the ammonia oxidizing bacteria. This pre-determined period of time is the period of time suitable to provide for sufficient polyphosphate loading to allow for the ammonia oxidizing bacteria to be stored for an extended period of time. The pre-determined period of time may be at least partially based on a period of time of about 0.2-10 times, 0.3-5 times, 0.5-3 times, 0.5-1.5 times, or 0.5 to 1 times the doubling time for the ammonia oxidizing bacteria. The pre-determined period of time may be at least partially based on a period of time of about one doubling time for the ammonia oxidizing bacteria. In some instances, the pre-determined period of time is between about 8 hours and 12 hours. In some instances, the pre-determined period of time is about 10 hours. In some instances, the pre-determined period of time is about 24 hours.

A purpose of the polyphosphate loading state may be to provide AOB with sufficient ammonia, ammonium ions, and/or urea, and O₂ such that ATP can be produced, but to deny them CO₂ and carbonate such that they are unable to use that ATP to fix CO₂ and instead use that ATP to generate polyphosphate which may be stored by the bacteria.

As used herein, the term "storage state" refers to autotrophic bacteria, *e*.*g*., ammonia oxidizing bacteria, in a state or in an environment, *e.g.,* a media, *e.g.,* a culture media, *e.g.,* a growth media, having a pH of about 7.4 or less (in some instances, the pH may be 7.6 or less). Levels of at least one of ammonia, ammonium ions, and urea are between about _1 and 1000 micromolar. Levels of trace materials are between about 0.1 and 100 micromolar. Levels of oxygen are between about 0 and 100% saturation (*e.g*., of media). Levels of carbon dioxide are between about 0 and 800 ppm. In certain aspects, levels of at least one of ammonia, ammonium ions, and urea are between about _10 and 100 micromolar. Levels of trace materials are between about 1 and 10 micromolar. Levels of oxygen are between about 0 and 100% saturation (*e.g*., of media). Levels of carbon dioxide are between about 0 and 400 ppm.

AOB are produced according to some instances of the present disclosure by generating AOB biomass during a growth state, then exposing the AOB to a polyphosphate loading state and then removing the media and resuspending the AOB in a buffer, *e.g.,* a storage buffer (*i.e.*, the storage state).

The ammonia oxidizing bacteria may remain in a "storage state" for a period of time, *e.g.,* a pre-determined period of time, for example, at least 1, 2, 3, 4, 5, 6, 7, days, 1, 2, 3, 4 weeks, 1, 2, 3, 4, 5, 6, 7, 8, 9, 10, 11, 12 months, 1, 2, 3, 4, or 5 years. In some instances, the ammonia oxidizing bacteria may remain in a storage state for at least about 6 months to about 1 year. Upon revival, the viability of the ammonia oxidizing bacteria is at least about 50%, 60%, 70%, 80%, 90%, or 100% of the viability as of the ammonia oxidizing bacteria prior to storage *e.g*., in a growth state). In some instances, the preparation of ammonia oxidizing bacteria may be prepared, such that no more than 10%, 20%, 30%, 40%, 50%, 60%, or 70% of the ability to oxidize NH₄⁺ is lost upon storage at selected conditions.

The time that it takes to revive the ammonia oxidizing bacteria from a storage state (or a polyphosphate loading state) may be a pre-determined period of time. For example, the pre-determined period of time may be less than about 75 hours, or less than about 72 hours. The pre-determined period of time may at least partially based on a period time of about 0.2-10 times, 0.3-5 times, 0.5-3 times, 0.5-1.5 times, or 0.5 to 1 times the doubling time for the ammonia oxidizing bacteria. The pre-determined period of time may be at least partially based on a period of time of about one doubling time for the ammonia oxidizing bacteria. The pre-determined period of time may be between about 8 hours and 12 hours. The pre-determined period of time may be about 10 hours. The pre-determined time may be less than about 75 hours, 72 hours, 70 hours, 68 hours, 65 hours, 60 hours, 55 hours, 50 hours, 45 hours, 40 hours, 35 hours, 30 hours, 25 hours, 20 hours, 15 hours, 10 hours, 5 hours, 4 hours, 3, hours, 2 hours, or 1 hour. The pre-determined period of time may be between about 5 minutes and 5 hours. The pre-determined period of time may be about 5-10 minutes, 10-15 minutes, 15-20 minutes, 20-25 minutes, 25-30 minutes, 30-45 minutes, 45-60 minutes, 60 minutes - 1.5 hours, 1.5 hours - 2 hours, 2 hours - 2.5 hours, 2.5 hours - 3 hours, 3 hours - 3.5 hours, 3.5 hours - 4 hours, 4 hours - 4.5 hours, 4.5 hours - 5 hours. In some instances, the pre-determined period of time may be about 2 hours.. The pre-determined period of time, *e.g*., may be the time it may take to achieve revival of the ammonia oxidizing bacteria, *e.g*., achieve viability of the ammonia oxidizing bacteria as compared to the viability of the bacteria prior to storage (*e.g*., in a growth state), *e.g*., at least about 10%, 20%, 30%, 40%, 50%, 60%, 70%, 80%, 90%, 95%, 97%, 98%, 99%, or 100% viability

Administered "in combination," as used herein, means that two (or more) different treatments are delivered to the subject during the course of the subject's affliction with the disorder, *e.g.,* the two or more treatments are delivered after the subject has been diagnosed with the disorder and before the disorder has been cured or eliminated. In some instances, the delivery of one treatment is still occurring when the delivery of the second begins, so that there is overlap. This is sometimes referred to herein as "simultaneous" or "concomitant" or "concurrent delivery". In other instances, the delivery of one treatment ends before the delivery of the other treatment begins. This is sometimes referred to herein as "successive" or "sequential delivery" or "consecutive delivery." In instances of either case, the treatment is more effective because of combined administration. For example, the second treatment is a more effective, *e.g*., an equivalent effect is seen with less of the second treatment, or the second treatment reduces symptoms to a greater extent, than would be seen if the second treatment were administered in the absence of the first treatment, or the analogous situation is seen with the first treatment. In some instances, delivery is such that the reduction in a symptom, or other parameter related to the disorder is greater than what would be observed with one treatment delivered in the absence of the other. The effect of the two treatments can be partially additive, wholly additive, or greater than additive (*i.e.*, synergistic). The delivery can be such that an effect of the first treatment delivered is still detectable when the second is delivered.

A "natural product" is or may comprise a product that may be at least partially derived from nature. It may be anything or comprise anything produced by a living organism, and may include organisms themselves. Natural products may include or comprise an entire organism, and part of an organism (*e.g*., a leaf of a plant), an extract from an organism, an organic compound from an organism, a purified organic compound from an organism. Natural products may be or comprise organic substances found and cells, including primary metabolites (amino acids, carbohydrates, and nucleic acids) and secondary metabolites (organic compounds found in a limited range of species, e.g., polyketides, fatty acids, terpenoids, steroids, phenylpropanoids, alkaloids, specialized amino acids and peptides, specialized carbohydrates). Natural products may be or comprise polymeric organic materials such as cellulose, lignin, and proteins.

Natural products may be or comprise products for commercial purposes, and may refer to cosmetics, dietary supplements, and foods produced from natural sources. Natural products may have pharmacological or biological activity that may be of therapeutic benefit, *e.g.*, in treating disease or conditions. Natural products may be included in traditional medicines, treatments for cosmetological purposes, and spa treatments. A natural product referred to herein may comprise any one or more of the components described as a natural product to be incorporated into a preparation or formulation comprising one or more other components, *e.g*., excipients. The preparation or formulation referred to as a natural product may comprise a natural product defined herein and one or more additional components or ingredients. Any of the compositions, preparations, or formulations discussed throughout this disclosure may be or comprise one or more natural products.

As used herein, "presence" or "level" may refer to a qualitative or quantitative amount of a component, *e.g*., any one or more of an ammonia oxidizing bacteria, ammonia, ammonium ions, urea, nitrite, or nitric oxide. The presence or level may include a zero value or a lack of presence of a component.

The terms "polypeptide", "peptide" and "protein" (if single chain) are used interchangeably herein to refer to amino acid polymers. The polymer may be linear or branched, it may comprise modified amino acids, and it may be interrupted by non-amino acids. The terms also encompass an amino acid polymer that has been modified; for example, disulfide bond formation, glycosylation, lipidation, acetylation, phosphorylation, or any other manipulation, such as conjugation with a labeling component. The polypeptide can be isolated from natural sources, can be a produced by recombinant techniques from a eukaryotic or prokaryotic host, or can be a product of synthetic procedures.

As used herein, the term "surfactant", includes compounds that may lower the surface tension, or interfacial tension, between two liquids or between a liquid and a solid. Surfactants may act as detergents, wetting agents, emulsifiers, foaming agents, and dispersants. Surfactants may include one or more of the following, alone, or in combination with those listed, or other surfactants or surfactant-like compounds: cocamidopropyl betaine (ColaTeric COAB), polyethylene sorbitol ester (*e*.*g*., Tween 80), ethoxylated lauryl alcohol (RhodaSurf 6 NAT), sodium laureth sulfate/lauryl glucoside/cocamidopropyl betaine (Plantapon 611 L UP), sodium laureth sulfate (e.g., RhodaPex ESB 70 NAT), alkyl polyglucoside (*e.g*., Plantaren 2000 N UP), sodium laureth sulfate (Plantaren 200), Dr. Bronner's Castile soap, Dr. Bronner's baby soap, Lauramine oxide (ColaLux Lo), sodium dodecyl sulfate (SDS), polysulfonate alkyl polyglucoside (PolySufanate 160 P), sodium lauryl sulfate (Stepanol-WA Extra K). and combinations thereof. Dr. Bronner's Castile soap and baby soap comprises water, organic coconut oil, potassium hydroxide, organic olive oil, organic fair deal hemp oil, organic jojoba oil, citric acid, and tocopherol.

As used herein, "transgenic" means comprising one or more exogenous portions of DNA. The exogenous DNA is derived from another organism, *e*.*g*., another bacterium, a bacteriophage, an animal, or a plant.

As used herein, "treatment of a disease or condition" refers to reducing the severity or frequency of at least one symptom of that disease or condition, compared to a similar but untreated patient. Treatment can also refer to halting, slowing, or reversing the progression of a disease or condition, compared to a similar but untreated patient. Treatment may comprise addressing the root cause of the disease and/or one or more symptoms.

As used herein a "therapeutically effective amount" refers to a dose sufficient to prevent advancement, or to cause regression of a disease or condition, or which is capable of relieving a symptom of a disease or condition, or which is capable of achieving a desired result. A therapeutically effective dose can be measured, for example, as a number of bacteria or number of viable bacteria (*e.g*., in CFUs) or a mass of bacteria (*e.g*., in milligrams, grams, or kilograms), or a volume of bacteria (*e.g*., in mm³).

As used herein, the term "viability" refers to the autotrophic bacteria's, *e.g.*, ammonia oxidizing bacteria's, ability to oxidize ammonia, ammonium, or urea to nitrite at a pre-determined rate. In some instances, the rate refers to the conversion of ammonium ions (NH₄⁺) (*e*.*g*., at about 200 mM) to nitrite (NO₂⁻) at a rate of at least 50, 75, 125, or 150 micromoles NO₂⁻ per minute, *e.g*., about 100-150, 75-175, 75-125, 100-125, 125-150, or 125-175 micromoles/minute, *e.g*., about 125 micromoles NO₂⁻ per minute.
"Growth media" or "AOB media," as referred to herein comprises the following components of Table 1 or Table 2:

**Table 1.**

| | Weight/Volume (in ∼ 1.5 L) | Final Concentration (in ∼ 1.5 L) |
|---|---|---|
| (NH₄)₂SO₄ (MW 132.14) | 4.95 g | 50 mM NH₄⁺ |
| KH₂PO₄ (MW 136.1) | 0.616 g | 3.0 mM |
| 1 M MgSO₄ | 1.137 ml | 0.76 mM |
| 1 M CaCl₂ | 0.3 ml | 0.2 mM |
| 30 mM FeCl₃ / 50mM EDTA | 0.5 ml | 10 µM / 16.7 µM |
| 50 mM CuSO₄ | 30 µl | 1.0 µM |
| Add 1400 ml ddH₂O to flask. Autoclave. Store at room temperature. | | |
| After autoclaving add: | | |
| Phosphate Buffer | 100 ml | 32 mM KH₂PO₄ / 2.7 mM NaH₂PO₄.H₂O |
| 5% Na₂CO₃ | 12 ml | 0.04% |

**Table 2.**

| | **Batch medium** Weight/Volume (1L) | **Feeding solution** Weight/Volume (1L) |
|---|---|---|
| | (Final concentration) | (Final concentration) |
| (NH₄)₂SO₄ (MW 132.14) | 3.3 g (50 mM NH₄⁺) | 13.2 g (200 mM NH₄⁺) |
| KH₂PO₄ (MW 136.1) | 1.23 g (9.0 mM) | 0.41 g (3.0 mM) |
| 1 M MgSO₄ | 0.758 ml | 0.758 ml |
| | (0.76 mM) | (0.76 mM) |
| 1 M CaCl₂ | 0.2 ml (0.2 mM) | 0.2 ml (0.2 mM) |
| 30 mM FeCl₃ / 50mM EDTA | 0.333 ml (10 µM / 16.7 µM) | 0.333 ml (10 µM / 16.7 µM) |
| 50 mM CuSO₄ | 20 µl (1.0 µM) | 20 µl (1.0 µM) |
| ddH₂O | 1000 ml | 1000 ml |
| Autoclave each solution and store at room temperature. | | |

### 2. Ammonia oxidizing bacteria (AOBs)

Autotrophic ammonia oxidizing bacteria, which may be referred to herein as AOBs or AOB, are obligate autotrophic bacteria as noted by Alan B. Hooper and A. Krummel at al. Alan B. Hooper, Biochemical Basis of Obligate Autotrophy in Nitrosomonas europaea, Journal of Bacteriology, Feb 1969, p. 776-779. Antje Krummel et al., Effect of Organic Matter on Growth and Cell Yield of Ammonia-Oxidizing Bacteria, Arch Microbiol (1982) 133: 50-54. These bacteria derive all metabolic energy only from the oxidation of ammonia to nitrite with nitric oxide (NO) as an intermediate product in their respiration chain and derive virtually all carbon by fixing carbon dioxide. They are incapable of utilizing carbon sources other than a few simple molecules.

Ammonia oxidizing bacteria (AOB) are widely found in the environment, and in the presence of ammonia, oxygen and trace metals will fix carbon dioxide and proliferate. AOB may be slow growing and toxic levels of ammonia may kill fish and other organisms before AOB can proliferate and reduce ammonia to non-toxic levels. Slow growth of AOB also may delay the health benefits of the NO and nitrite the AOB produce when applied to the skin.

Supplementing the aquarium, skin, or process with sufficient viable AOB grown and stored for that purpose is desired. AOB do not form spores, so storage in the dry state with high viability is difficult, and storage in the wet state leaves them metabolically active.

Decay of nitrifying capacity during storage of AOB for wastewater treatment has been studied, as for example (Munz G, Lubello C, Oleszkiewicz JA. Modeling the decay of ammonium oxidizing bacteria. Water Res. 2011 Jan; 45(2): 557-64. Oi: 10.1016/j.watres.2010.09.022.)

Growth, prolonged storage, and restoration of activity of *Nitrosomonas* is discussed by Cassidy et al. (U.S. 5,314,542) where they disclose growing *Nitrosomonas,* removing toxic waste products, storing in sterile water of appropriate salinity for periods of time up to one year, and then reviving by adding buffer (CaCO₃) and 200 ppm, of ammonium, which reviving takes 72 hours.

The present disclosure provides that if AOB are kept under conditions of low carbon dioxide but with sufficient oxygen and ammonia, where they accumulate polyphosphate for a period of about one doubling time (∼10 hours), then they accumulate sufficient polyphosphate to greatly extends their storage viability, storage time and accelerate their revival both with and without addition of buffer and ammonia as disclosed by Cassidy et. al.

As obligate autotrophs, AOB synthesize protein via the fixing of CO₂ using the energy and reducing equivalents generated by the oxidation of ammonia to nitrite. Growth requires ammonia, oxygen, minerals and carbon dioxide.

*Nitrosomonas* may exist in several metabolic states, according to "Polyphosphate and Orthophosphate Content of Nitrosomonas europaea as a Function of Growth" by K.R. Terry and A.B. Hooper, Journal of Bacteriology, July 1970, p. 199-206, Vol. 103, No. I.

The AOBs contemplated in this disclosure may comprise mutations relative to wild-type AOBs. These mutations may, *e.g*., occur spontaneously, be introduced by random mutagenesis, or be introduced by targeted mutagenesis. For instance, the AOBs may lack one or more genes or regulatory DNA sequences that wild-type AOBs typically comprise. The AOBs may also comprise point mutations, substitutions, insertions, deletions, and/or rearrangements relative to the sequenced strain or a wild-type strain. The AOBs may be a purified preparation of optimized AOBs.

In certain embodiments, the AOBs are transgenic. For instance, it may comprise one or more genes or regulatory DNA sequences that wild-type ammonia oxidizing bacteria lacks. More particularly, the ammonia oxidizing bacteria may comprise, for instance, a reporter gene, a selective marker, a gene encoding an enzyme, or a promoter (including an inducible or repressible promoter). In some embodiments the additional gene or regulatory DNA sequence is integrated into the bacterial chromosome; in some embodiments the additional gene or regulatory DNA sequence is situated on a plasmid.

In some embodiments, the AOBs differ by at least one nucleotide from naturally occurring bacteria. For instance, the AOBs may differ from naturally occurring bacteria in a gene or protein that is part of a relevant pathway, *e.g.,* an ammonia metabolism pathway, a urea metabolism pathway, or a pathway for producing nitric oxide or nitric oxide precursors. More particularly, the AOBs may comprise a mutation that elevates activity of the pathway, *e.g.,* by increasing levels or activity of an element of that pathway.

The above-mentioned mutations can be introduced using any suitable technique. Numerous methods are known for introducing mutations into a given position. For instance, one could use site-directed mutagenesis, oligonucleotide-directed mutagenesis, or site-specific mutagenesis. Non-limiting examples of specific mutagenesis protocols are described in, *e.g*., Mutagenesis, pp. 13.1-13.105 (Sambrook and Russell, eds., Molecular Cloning A Laboratory Manual, Vol. 3, 3.sup.rd ed. 2001). In addition, non-limiting examples of well-characterized mutagenesis protocols available from commercial vendors include, without limitation, Altered Sites.RTM. II in vitro Mutagenesis Systems (Promega Corp., Madison, Wis.); Erase-a-Base.RTM. System (Promega, Madison, Wis.); GeneTailor.TM. Site-Directed Mutagenesis System (Invitrogen, Inc., Carlsbad, Calif.); QuikChange.RTM. II Site-Directed Mutagenesis Kits (Stratagene, La Jolla, Calif.); and Transformer.TM. Site-Directed Mutagenesis Kit (BD-Clontech, Mountain View, Calif.).

In some embodiments of the disclosure, the ammonia oxidizing bacteria may be axenic. The preparation, *e.g*., formulation, *e.g*., composition) of ammonia oxidizing bacteria may comprise, consist essentially of, or consist of axenic ammonia oxidizing bacteria. The ammonia oxidizing bacteria may be from a genus selected from the group consisting of *Nitrosomonas, Nitrosococcus, Nitrosospira, Nitrosocystis, Nitrosolobus, Nitrosovibrio,* and combinations thereof.

In some embodiments, the preparation of ammonia oxidizing bacteria may comprise a concentration or amount of ammonia oxidizing bacteria in order to at least partially treat a condition or disease. The preparation of ammonia oxidizing bacteria may comprise a concentration or amount of ammonia oxidizing bacteria in order to alter, *e.g*., reduce or increase, an amount, concentration or proportion of a bacterium, or genus of bacteria, on a surface, *e.g*., a skin surface. The bacteria may be non-pathogenic or pathogenic, or potentially pathogenic.

In some embodiments, the preparation of ammonia oxidizing bacteria may comprise between about 10⁸ to about 10¹⁴ CFU/L. The preparation may comprise at least 10⁸, 10⁹, 10¹⁰, 10¹¹, 2 x 10¹¹, 5 x 10¹¹, 10¹², 2 x 10¹², 5 x 10¹², 10¹³, 2 x 10¹³, 5 x 10¹³, or 10¹⁴; or about 10⁸-10⁹, 10⁹-10¹⁰, 10¹⁰-10¹¹, 10¹¹-10¹², 10¹²-10¹³, or 10¹³-10¹⁴ CFU/L. In some embodiments, the preparation may comprise at least 10⁸, 10⁹, 10¹⁰, 10¹¹, 2 x 10¹¹, 5 x 10¹¹, 10¹², 2 x 10¹², 5 x 10¹², 10¹³, 2 x 10¹³, 5 x 10¹³, or 10¹⁴; or about 10⁸-10⁹, 10⁹-10¹⁰, 10¹⁰-10¹¹, 10¹¹-10¹², 10¹²-10¹³, or 10¹³-10¹⁴ CFU/ml.

In certain aspects, the preparation may comprise between about 1 x 10⁹ CFU to about 10 x 10⁹ CFU. In certain aspects, the preparation may comprise between about 1 x 10⁹ CFU/L to about 10 x 10⁹ CFU/L.

In some embodiments, the preparation of ammonia oxidizing bacteria may comprise between about 0.1 milligrams (mg) and about 1000 mg of ammonia oxidizing bacteria. In certain aspects, the preparation may comprise between about 50 mg and about 1000 mg of ammonia oxidizing bacteria. The preparation may comprise between about 0.1-0.5 mg, 0.2-0.7 mg, 0.5-1.0 mg, 0.5-2 mg, 0.5-5 mg, 2.5-5 mg, 2.5-7.0 mg, 5.0-10 mg, 7.5-15 mg, 10-15 mg, 15-20 mg, 15-25 mg, 20-30 mg, 25-50 mg, 25-75 mg, 50-75 mg, 50-100 mg, 75-100 mg, 100-200 mg, 200-300 mg, 300-400 mg, 400-500 mg, 500-600 mg, 600-700 mg, 700-800 mg, 800-900 mg, 900-1000 mg, 100-250 mg, 250-500 mg, 100-500 mg, 500-750 mg, 750-1000 mg, or 500-1000 mg.

In some embodiments, the preparation of ammonia oxidizing bacteria may comprise a mass ratio of ammonia oxidizing bacteria to an excipient, *e.g.,* a pharmaceutically acceptable excipient or a cosmetically acceptable excipient in a range of about 0.1 grams per liter to about 1 gram per liter. The preparation may comprise a mass ratio of ammonia oxidizing bacteria to an excipient in a range of about 0.1-0.2, 0.2-0.3, 0.1-0.5, 0.2-0.7, 0.5-1.0, or 0.7-1.0 grams per liter.

In some embodiments, the preparation of ammonia oxidizing bacteria may comprise, consist essentially of, or consist of ammonia oxidizing bacteria in a buffer solution comprising, consisting essentially of, or consisting of disodium phosphate and magnesium chloride, for example, 50 mM Na₂HPO₄ and 2 mM MgCl₂.

The preparation may comprise a volume of between about 0.1 and about 100 fluid ounces, about 0.2 and about 50 fluid ounces, about 0.5 and about 25 fluid ounces, about 1.0 and about 10 fluid ounces, about 2.0 and about 7 fluid ounces, about 3 and about 5 fluid ounces. In some embodiments, the preparation may comprise a volume of about 3.4 fluid ounces.

The preparation may be provided in a container constructed to contain between about 0.1 and about 100 fluid ounces, about 0.2 and about 50 fluid ounces, about 0.5 and about 25 fluid ounces, about 1.0 and about 10 fluid ounces, about 2.0 and about 7 fluid ounces, about 3 and about 5 fluid ounces. In some embodiments, the preparation is a container constructed to contain about 3.4 fluid ounces. The container may be a one-chamber container, or any other container disclosed herein.

In some embodiments, the preparation of ammonia oxidizing bacteria may be in a growth state. A growth state may be provided by exposing ammonia oxidizing bacteria to an environment that may promote growth. The growth state may be a state, *e.g*., ammonia oxidizing bacteria in an environment that allows immediate availability of ammonia oxidizing bacteria to convert ammonium ions (NH₄⁺) to nitrite (NO₂⁻). The growth state may comprise providing ammonia oxidizing bacteria in an environment having a pH of greater than about 7.6. The growth state may also comprise providing ammonia oxidizing bacteria in an environment having ammonia, ammonium ions, and/or urea, trace minerals and sufficient oxygen and carbon dioxide, as described above in Section 1.

In some embodiments, the preparation of ammonia oxidizing bacteria may be in a polyphosphate loading state, wherein the state or the environment, *e.g*., a media, *e.g*., a culture media, *e.g.,* a growth media, may have a pH of less than about 7.4. Levels of components are selected to provide AOB with ammonia and oxygen such that ATP can be produced, but to deny them carbon dioxide and carbonate such that they are unable to use that ATP to fix carbon dioxide and instead use that ATP to generate polyphosphate which may be stored.

In some embodiments, the preparation of ammonia oxidizing bacteria may be in a storage state. A storage state may be defined as ammonia oxidizing bacteria in an environment in which they may be stored to be later revived. The storage state may be a state, *e.g*., ammonia oxidizing bacteria in an environment that allows availability of ammonia oxidizing bacteria after being revived, *e.g*., after being place in an environment promoting a growth state for a pre-determined period of time.

The storage state may comprise providing ammonia oxidizing bacteria in an environment having a pH of less than about 7.4. The storage state may also comprise providing ammonia oxidizing bacteria in an environment having ammonia, ammonia ions, and/or urea, trace minerals, oxygen, and low concentrations of carbon dioxide, as described above in Section 1.

Storage may also be accomplished by storing at 4°C for up to several months. The storage buffer in some embodiments may comprise 50 mM Na₂HPO₄ - 2 mM MgCl₂ (pH 7.6).

In some embodiments, ammonia oxidizing bacteria may be cyropreserved. A 1.25 ml of ammonia oxidizing bacteria mid-log culture may be added to a 2 ml cryotube and 0.75 ml of sterile 80% glycerol. Tubes may be shaken gently, and incubate at room temperature for 15 min to enable uptake of the cryoprotective agents by the cells. The tubes may be directly stored in a -80°C freezer for freezing and storage.

For resuscitation of cultures, frozen stocks may be thawed on ice for 10 - 20 minutes, and then centrifuged at 8,000 x g for 3 minutes at 4°C. The pellet may be washed by suspending it in 2 ml AOB medium followed by another centrifugation at 8,000 x g for 3 minutes at 4°C to reduce potential toxicity of the cryoprotective agents. The pellet may be resuspended in 2 ml of AOB medium, inoculated into 50 ml of AOB medium containing 50 mM NH₄⁺, and incubated in dark at 30°C by shaking at 200 rpm.

In some embodiments, the preparation of ammonia oxidizing bacteria may comprise ammonia oxidizing bacteria in a storage state and/ or ammonia oxidizing bacteria in a polyphosphate loading state, and/or ammonia oxidizing bacteria in a growth state.

Without wishing to be bound by theory, by maintaining ammonia oxidizing bacteria under conditions or in an environment of low carbon dioxide, with sufficient oxygen and ammonia, they may accumulate polyphosphate for a pre-determined period, *e.g.,* for a period of about one doubling time, *e.g.,* for about 8-12 hours, *e.g.,* for about 10 hours. The ammonia oxidizing bacteria may accumulate sufficient polyphosphate to extend their storage viability, storage time, and accelerate their revival. This may occur with or without the addition of buffer and ammonia.

The presence of sufficient stored polyphosphate may allow the ammonia oxidizing bacteria the ATP resources to maintain metabolic activity even in the absence of ammonia and oxygen, and to survive insults that would otherwise be fatal.

The process of oxidation of ammonia to generate ATP has two steps. The first step is the oxidation of ammonia to hydroxylamine by ammonia monoxoygenase (Amo), followed by the conversion of hydroxylamine to nitrite by hydroxylamine oxidoreductase (Hao). Electrons from the second step (conversion of hydroxylamine to nitrite) are used to power the first step (oxidation of ammonia to hydroxylamine).

If an ammonia oxidizing bacteria does not have hydroxylamine to generate electrons for Amo, then hydroxylamine is not available for Hao. For example, acetylene irreversibly inhibits the enzyme crucial for the first step in the oxidation of ammonia to nitrite, the oxidation of ammonia to hydroxylamine. Once AOB are exposed to acetylene, Amo is irreversibly inhibited and new enzyme must be synthesized before hydroxylamine can be generated. In a normal consortium biofilm habitat, AOB may share and receive hydroxylamine form other AOB (even different strains with different susceptibilities to inhibitors) and so the biofilm tends to be more resistant to inhibitors such as acetylene than an individual organism. AOB can use stored polyphosphate to synthesize new Amo, even in the absence of hydroxylamine.

Any embodiment, preparation, composition, or formulation of ammonia oxidizing bacteria discussed herein may comprise, consist essentially of, or consist of optionally axenic ammonia oxidizing bacteria.

### 3. Methods of producing Ammonia Oxidizing Bacteria

Methods of culturing various ammonia oxidizing bacteria, *e.g*., *Nitrosomonas* species are known in the art. Ammonia oxidizing bacteria may be cultured, for example, using media described in Table 1 or Table 2, above.

Ammonia oxidizing bacteria may be grown, for example, in a liquid culture or on plates. Suitable plates include 1.2% R2A agar, 1.2% agar, 1.2% agarose, and 1.2% agarose with 0.3 g/L pyruvate.

In some instances, ammonia oxidizing bacteria may be cultured in organic free media. One advantage of using organic free media is that it lacks substrate for heterotrophic bacteria to metabolize except for that produced by the autotrophic bacteria. Another advantage of using the as-grown culture is that substantial nitrite accumulates in the culture media, and this nitrite is also inhibitory of heterotrophic bacteria and so acts as a preservative during storage.

In some instances, an ammonia oxidizing bacteria with improved, e.g. optimized, properties is produced by an iterative process of propagation and selecting for desired properties. In some instances, the selection and propagation are carried out simultaneously. In some instances, the selection is carried out in a reaction medium (*e.g*., complete *N. europaea* medium) comprising 50 mM, 75 mM, 100 mM, 125 mM, 150 mM, 175 mM, 200 mM, 225 mM, 250 mM, 275 mM, or 300 mM NH₄⁺, *e.g.*, at least 200 mM NH₄⁺. In some instances, the period of propagation and/or selection is at least 1, 2, 3, or 6 months. In instances, the period of propagation and/or selection is at least 1, 2, 4, 6, 8, or 10 years.

In some aspects, the ammonia oxidizing bacteria are manufactured on a commercial scale. In some instances, commercial scale refers to a liquid culturing method with a culture medium volume of at least 10,000, 20,000, 30,000, 50,000, or 100,000 liters (L). In some instances, the bacteria are produced in a bioreactor. The bioreactor may maintain the bacteria at a constant temperature, *e.g.*, about 26-30 degrees Celsius using, for example a thermal jacket for insulation, a temperature sensor, and a heating or cooling element. The bioreactor may have an apparatus for stirring the culture to improve distribution of nutrients like ammonia, urea, oxygen, carbon dioxide, and various minerals. The bioreactor may also have an inlet tube for addition of new medium, and an outlet tube for collection of cells. The bioreactor may also have an aerator for distributing oxygen and/or carbon dioxide to the culture. The bioreactor may be, *e.g.,* a batch reactor, a fed batch reactor, or a continuous reactor. In some instances, commercial scale production of ammonia oxidizing bacteria yields a batch of 1,000 to 100,000 L per day at about 10¹² CFU / liter. The commercial scale production may yield *e.g.,* a batch of 1,000-5,000, 5,000-10,000, 10,000-50,000, or 50,000-100,000 L/day. The commercial scale production may yield *e.g.,* a batch of 1,000-5,000, 5,000-10,000, 10,000-50,000, or 50,000-100,000 L per batch. In some instances, the yield is at a concentration of at least 10⁸, 10⁹, 10¹⁰, 10¹¹, 2 x 10¹¹, 5 x 10¹¹, or 10¹², or about 10¹⁰-10¹¹, 10¹¹-10¹², 10¹²-10¹³, or 10¹³-10¹⁴ CFU/L. In some instances, the yield is at a concentration of at least 10⁸, 10⁹, 10¹⁰, 10¹¹, 2 x 10¹¹, 5 x 10¹¹, 10¹², 2 x 10¹², 5 x 10¹², 10¹³, 2 x 10¹³, 5 x 10¹³, or 10¹⁴; or about 10⁸-10⁹, 10⁹-10¹⁰, 10¹⁰-10¹¹, 10¹¹-10¹², 10¹²-10¹³, or 10¹³-10¹⁴ CFU/ml.

In some instances, typically including commercial scale production, quality control (QC) testing steps are carried out. The general steps of QC may comprise, 1) culturing ammonia oxidizing bacteria, 2) performing a testing step on the culture or an aliquot thereof, and 3) obtaining a value from the testing step, and optionally: 4) comparing the obtained value to a reference value or range of acceptable values, and 5) if the obtained value meets the acceptable reference value or range, then classifying the culture as acceptable, and if the obtained value does not meet the acceptable reference value or range, then classifying the culture as unacceptable. If the culture is classified as acceptable, the culture may, *e.g.,* be allowed to continue growing and/or may be harvested and added to a commercial product. If the culture is classified as unacceptable, the culture may, *e.g.,* be safely disposed of or the defect may be remedied.

The testing step may comprise measuring the optical density (OD) of the culture. OD is measured in a spectrophotometer, and provides information on the amount of light transmitted through the sample as distinguished from light absorbed or scattered. In some instances, the OD600 (*e.g*., optical density of light with a wavelength of 600 nm) may be determined. This measurement typically indicates the concentration of cells in the medium, where a higher optical density corresponds to a higher cell density.

The testing step may comprise measuring the pH of the culture. The pH of an ammonia oxidizing bacteria culture indicates the rate of nitrogen oxidation, and can also indicate whether the culture comprises a contaminating organism. pH may be measured using, *e.g*., a pH-sensing device comprising a electrode (such as a hydrogen electrode, quinhydron-Electrode, antimony electrode, glass electrode), a pH-sensing device comprising a semiconductor, or a color indicator reagent such as pH paper.

In certain instances, producing the ammonia oxidizing bacteria comprises carrying out various quality control steps. For instance, one may test the medium in which the ammonia oxidizing bacteria is grown, *e.g.,* to determine whether it has an appropriate pH, whether it has a sufficiently low level of waste products, and/or whether it has a sufficiently high level or nutrients. One may also test for the presence of contaminating organisms. A contaminating organism is typically an organism other than ammonia oxidizing bacteria, for instance an organism selected from *Microbacterium* sp., *Alcaligenaceae* bacterium, *Caulobacter* sp., *Burkodelia multivorans*, *Escherichia coli*, *Klebsiella pneumoniae*, and *Staphylococcus aureus.* One may test for contaminants by, *e.g.,* extracting DNA, amplifying it, and sequencing a conserved gene such as 16S rRNA. One may also test for contaminants by plating culture on agar plates and observing colony morphology. Ammonia oxidizing bacteria typically forms red colonies, so non-red colonies are often indicative of contaminating organisms.

### 4. Methods of Testing Product for Ammonia Oxidizing Bacteria and Quality Control Methods

Methods of evaluating a preparation for the level of ammonia oxidizing bacteria according to the present invention are set out in the appended claims.

The ammonia oxidizing bacteria may be of the genus selected from the group consisting of *Nitrosomonas*, *Nitrosococcus*, *Nitrosospira*, *Nitrosocystis*, *Nitrosolobus*, *Nitrosovibrio*, and combinations thereof.

The preparation may be a cosmetic product or a therapeutic product, or may be in the process of being prepared or manufactured as a cosmetic product or a therapeutic product. The preparation may be disposed in or provided as a cosmetic product or a therapeutic product. The preparation may be supplied to, *e.g.,* a manufacturer, healthcare company, or consumer product company that may use the preparation or convert the preparation into a therapeutic product or a consumer product.

The methods of evaluating a preparation may be provided to evaluate, *e.g.,* a natural product, a product that has been manufactured, a product that may naturally contain ammonia oxidizing bacteria, or one in which ammonia oxidizing bacteria have been added or will be added.
The method of the invention comprises measuring an amount or a concentration of ammonia oxidizing bacteria in the product to provide a value. This may be accomplished by measuring the pH of the preparation. The pH of an ammonia oxidizing bacteria preparation may indicate the rate of nitrogen oxidation, and may also indicate whether the preparation comprises a contaminating organism. pH may be measured using, *e.g*., a pH-sensing device comprising a electrode (such as a hydrogen electrode, quinhydron-Electrode, antimony electrode, glass electrode), a pH-sensing device comprising a semiconductor, or a color indicator reagent such as pH paper. In some embodiments, a sample of the preparation may be cultured to provide an ammonia oxidizing culture, that may then be tested by measuring the pH of the culture.

Measuring an amount or a concentration of ammonia oxidizing bacteria in the product to provide a value may be accomplished by measuring the rate of oxidation of ammonia, ammonium ions, and/or urea to provide nitrite, *e.g*., a rate of conversion of ammonia, ammonium ions, and/or urea to nitrite, or measuring a level of nitrite in a sample, such as with the use of the Griess Reagent, as would be known to one of ordinary skill in the art. Measuring the presence or level of nitrite in the preparation may also accomplished to provide a value. In some embodiments, measuring an amount or concentration of protein in the product may also be accomplished to provide a value. The amount or concentration of total protein in a sample may be used to calculate the concentration of ammonia oxidizing bacteria.

In some embodiments, the methods of the present invention comprise classifying the preparation as requiring addition of ammonia oxidizing bacteria, or as not requiring addition of ammonia oxidizing bacteria. This may be accomplished by evaluating the value measured, as discussed above, to determine whether it is within or outside a pre-determined range of values.

The value may be compared to a range of values corresponding to the pre-determined range of values, *e.g*., a range of amounts of ammonia oxidizing bacteria, *e.g*., a range of concentrations of ammonia oxidizing bacteria. It then may be determined if the value is in the range of values corresponding to the pre-determined range of values. If the value is in the range of values corresponding to the pre-determined range of values, the product may be classified as accepted. This may mean that the product may be suitable for manufacturing, packaging, placing into commerce, supplying or selling to, *e.g*., a manufacturer, a healthcare company, consumer product company, *e.g*., a cosmetic company or beauty supply company, a health care provider, or a consumer.

If the value is outside the range of values corresponding to the pre-determined range of values, the product may be classified as not accepted. If the value is outside the range of values corresponding to the pre-determined range of amounts or concentrations of ammonia oxidizing bacteria, the preparation may be further processed or discarded. If the preparation is further processed, the preparation may be diluted, if the amount or concentration of ammonia oxidizing bacteria is higher than the pre-determined range of amounts or concentrations of ammonia oxidizing bacteria. If the preparation is further processed, additional components may be added, for example, if the value is outside the range of values corresponding to the pre-determined range of values, *e.g.*, below the range of values corresponding to the pre-determined range.

In some embodiments in which the value is outside the range of values corresponding to the pre-determined range of values, an amount or concentration of ammonia oxidizing bacteria may be added to the preparation. In other embodiments, addition of one or more of ammonia, ammonium ions, and urea may be performed. In some embodiments, a combination of one or more of ammonia oxidizing bacteria, ammonia, ammonium ions, and urea may be added.

Further evaluation may be performed to determine if the preparation may be classified as accepted, or not accepted, as described above.

The product may be further evaluated to determine if contaminating organisms, *e.g.*, pathogenic organisms, are present. This may be accomplished by testing the pH of the preparation. Pathogenic organisms may include *Staphylococcus aureus* (*S. aureus*), *Psuedonomas aeruginosa* (*P. aeruginosa*), *Streptococcus pyogenes* (*S. pyogenes*), *Acinetobacter baumannii* (*A. baumannii*), *Propionibacteria*, and *Stenotrophomonas*, and combinations thereof. This may also be accomplished by ingsin bioburden tests, e.g., plating a sample of the preparation on LB agar plates and culturing them at 30-37°C for a period of time, *e.g*., about 24 hours or more, to determine the presence of pathogenic organisms. This may also be accomplished by using 15S rRNA sequencing to determine pathogens that may be present.

In some embodiments, a sample from the product may be selected and testing may be conducted on the sample.

The range of values corresponding to a pre-determined range of concentrations of ammonia oxidizing bacteria may be between about 10⁸ CFU/L to about 10¹⁴ CFU/L. In certain aspects, the range of values corresponding to the pre-determined range of amounts or concentrations of ammonia oxidizing bacteria is less than about 10^{8,} or between about 10⁸ - 10⁹, 10⁹ - 10¹⁰, 10¹⁰ - 10¹¹, 10¹¹ - 10¹², 10¹² - 10¹³, or 10¹³ - 10¹⁴ CFU/L.

The range of values corresponding to a pre-determined range of concentrations of ammonia oxidizing bacteria may be between about 10⁸ CFU/ml to about 10¹⁴ CFU/ml. In certain aspects, the range of values corresponding to the pre-determined range of amounts or concentrations of ammonia oxidizing bacteria is less than about 10^{8,} or between about 10⁸ - 10⁹, 10⁹ - 10¹⁰, 10¹⁰ - 10¹¹, 10¹¹ - 10¹², 10¹² - 10¹³, or 10¹³ - 10¹⁴ CFU/ml.

In certain aspects, the preparation may comprise between about 1 x 10⁹ CFU to about 10 x 10⁹ CFU.

The range of values corresponding to a pre-determined range of amounts of ammonia oxidizing bacteria may comprise between about 0.1 milligrams (mg) and about 1000 mg of ammonia oxidizing bacteria. In certain aspects, the preparation may comprise between about 50 mg and about 1000 mg of ammonia oxidizing bacteria. The range may comprise between about 0.1-0.5 mg, 0.2-0.7 mg, 0.5-1.0 mg, 0.5-2 mg, 0.5-5 mg, 2.5-5 mg, 2.5-7.0 mg, 5.0-10 mg, 7.5-15 mg, 10-15 mg, 15-20 mg, 15-25 mg, 20-30 mg, 25-50 mg, 25-75 mg, 50-75 mg, 50-100 mg, 75-100 mg, 100-200 mg, 200-300 mg, 300-400 mg, 400-500 mg, 500-600 mg, 600-700 mg, 700-800 mg, 800-900 mg, 900-1000 mg, 100-250 mg, 250-500 mg, 100-500 mg, 500-750 mg, 750-1000 mg, or 500-1000 mg.

The method of evaluating of the present invention may comprise evaluating the preparation from a first batch, *e.g*., a first batch for commercial release. The preparation from a second batch may be evaluated, *e.g*., a second batch for commercial release. One or more additional batches, *e.g*., a third, fourth, fifth, sixth, seventh, eighth, ninth, tenth, fifteen twentieth, hundredth, or more, batches may be evaluated, *e.g*., for commercial release. Multiple batches may be evaluated, and additional batches may be evaluated at pre-determined time or batch intervals in order to, *e.g*., evaluate the consistency of preparations over time, and over manufacturing batches, which also may be referred to as lots.

The method of the present invention may comprise evaluating the preparation from a plurality of batches to determine if each batch within the plurality meets the pre-determined range of amounts or concentrations of ammonia oxidizing bacteria.

The batches may comprise releasing the plurality of batches. The plurality of batches, *e.g*., at least one batch, may be commercially released to, *e.g*., a manufacturer, a healthcare company, consumer product company, *e.g.*, a cosmetic company or beauty supply company, a health care provider, or a consumer.

The batches may be prepared simultaneously or consecutively. Simultaneously may mean that the preparation of one batch is still occurring when the preparation of the second batch begins, so that there is overlap. This may be referred to as "simultaneous," "concomitant," or "concurrent" preparation. Consecutively may mean that the preparation of one batch ends before the preparation of the second batch. This may be referred to herein as "successive," "sequential," or "consecutive" preparation.

Compositions may be provided, *e.g*., preparations or formulations, from the methods of evaluating as described above. The compositions may comprise one or more excipients as described throughout this disclosure, and may be suitable for one of topical, nasal, pulmonary, and gastrointestinal administration. The compositions may be substantially free of other organisms. They may be disposed in, or provided as a powder, cosmetic, cream, stick, aerosol, salve, wipe, or bandage, and them may further comprise a moisturizing agent, deodorizing agent, scent, colorant, insect repellant, cleansing agent, or UV-blocking agent, as described herein. The compositions may comprise an organism selected from the group consisting of *Lactobacillus*, *Streptococcus*, *Bifidobacter*, and combinations thereof.

The compositions described herein, *e.g*., provided by the methods of evaluating, may be useful as a treatment or prevention of a skin disorder, a treatment or prevention of a disease or condition associated with low nitrite levels, a treatment or prevention of body odor, a treatment to supply nitric oxide to a subject, or a treatment to inhibit microbial growth.

They may be useful for treatment of at least one of HIV, dermatitis, infection in an ulcer, e.g., venous ulcer, e.g., leg ulcer, e.g., venous leg ulcer, e.g. infection in a diabetic foot ulcer, atopic dermatitis, acne, e.g., acne vulgaris, eczema, contact dermatitis, allergic reaction, psoriasis, uticaria, rosacea, skin infections, vascular disease, vaginal yeast infection, a sexually transmitted disease, heart disease, atherosclerosis, baldness, leg ulcers secondary to diabetes or confinement to bed, angina, particularly chronic, stable angina pectoris, ischemic diseases, congestive heart failure, myocardial infarction, ischemia reperfusion injury, laminitis, hypertension, hypertrophic organ degeneration, Raynaud's phenomenon, fibrosis, fibrotic organ degeneration, allergies, autoimmune sensitization, end stage renal disease, obesity, impotence, pneumonia, primary immunodeficiency, epidermal lysis bulosa, or cancer.

In certain aspects the compositions described herein may be useful for treatment of at least one of an infection in a diabetic foot ulcer, atopic dermatitis, acne, e.g., acne vulgaris, eczema, psoriasis, uticaria, rosacea, and skin infections.

The compositions described herein, *e.g*., provided by the methods of evaluating, may comprise, be provided as, or disposed in at least one of a baby product, *e.g.,* a baby shampoo, a baby lotion, a baby oil, a baby powder, a baby cream; a bath preparation, *e.g*., a bath oil, a tablet, a salt, a bubble bath, a bath capsule; an eye makeup preparation, *e.g*., an eyebrow pencil, an eyeliner, an eye shadow, an eye lotion, an eye makeup remover, a mascara; a fragrance preparation, *e.g*., a colognes, a toilet water, a perfume, a powder (dusting and talcum), a sachet; hair preparations, *e.g*., hair conditioners, hair sprays, hair straighteners, permanent waves, rinses, shampoos, tonics, dressings, hair grooming aids, wave sets; hair coloring preparations, *e.g*., hair dyes and colors, hair tints, coloring hair rinses, coloring hair shampoos, hair lighteners with color, hair bleaches; makeup preparations, *e.g*., face powders, foundations, leg and body paints, lipstick, makeup bases, rouges, makeup fixatives; manicuring preparations, *e.g*., basecoats and undercoats, cuticle softeners, nail creams and lotions, nail extenders, nail polish and enamel, nail polish and enamel removers; oral hygiene products, *e.g*., dentrifices, mouthwashes and breath fresheners; bath soaps and detergents, deodorants, douches, feminine hygiene deodorants; shaving preparations, *e.g*., aftershave lotions, beard softeners, talcum, preshave lotions, shaving cream, shaving soap; skin care preparations, *e.g*., cleansing, depilatories, face and neck, body and hand, foot powders and sprays, moisturizing, night preparations, paste masks, skin fresheners; and suntan preparations, *e.g*., gels, creams, and liquids, and indoor tanning preparations.

The compositions described herein, *e.g*., provided by the methods of evaluating, may comprise, be provided as, or disposed in at least one of a baby product, *e.g.,* a baby shampoo, a baby lotion, a baby oil, a baby powder, a baby cream; a bath preparation, *e.g*., a bath oil, a tablet, a salt, a bubble bath, a bath capsule; a powder (dusting and talcum), a sachet; hair preparations, *e.g*., hair conditioners, rinses, shampoos, tonics, face powders, cuticle softeners, nail creams and lotions, oral hygiene products, mouthwashes, bath soaps, douches, feminine hygiene deodorants; shaving preparations, *e.g*., aftershave lotions, skin care preparations, *e.g*., cleansing, face and neck, body and hand, foot powders and sprays, moisturizing, night preparations, paste masks, skin fresheners; and suntan preparations, *e.g*., gels, creams, and liquids.

### 5. Methods of Evaluating a Subject

Methods of evaluating a subject are disclosed herein. The methods may comprise evaluating a subject for application of ammonia oxidizing bacteria. This may comprise a self-evaluation in which a subject evaluates their own condition, *e*.*g*., condition of the skin, or their health condition, and evaluate whether to administer or apply ammonia oxidizing bacteria to their self. This may also comprise evaluation of a subject by another, *e.g*., a healthcare professional, to evaluate the subject's condition, *e.g*., condition of the skin, or their health condition, and evaluate whether to administer or apply ammonia oxidizing bacteria to the subject, or advise the subject or another individual to administer or apply ammonia oxidizing bacteria to the subject. The methods may be used to determine if the subject qualifies for application of ammonia oxidizing bacteria.

The methods may comprise assessing the subject for a skin condition. Responsive to the assessing, one of the following may be performed: (1) classifying the subject as requiring ammonia oxidizing bacteria; and (2) administering ammonia oxidizing bacteria. This may provide a method for determining if a subject qualifies for application of ammonia oxidizing bacteria. The subject may perform the step of assessing, or another individual may peform the step of assessing. In some instances, the subject may be classified as not requiring ammonia oxidizing bacteria. In these instances, the subject may not be administered ammonia oxidizing bacteria.

The skin disorder or skin condition that may be assessed may be selected from the group consisting of dermatitis, general itchiness, infection in an ulcer, e.g., venous ulcer, e.g., leg ulcer, e.g., venous leg ulcer, e.g. infection in a diabetic foot ulcer, atopic dermatitis, acne, e.g., acne vulgaris, eczema, contact dermatitis, allergic reaction, psoriasis, uticaria, rosacea, skin infections, leg ulcers secondary to diabetes or confinement to bed, epidermal lysis bulosa, and allergies.

The method may comprise obtaining a sample from a surface of the subject. This may involve any method of obtaining a sample from a surface of the subject, *e.g*., contacting the surface of the subject, *e.g.*, the skin, with a wipe, cloth, cotton ball, cotton swab, gauze, towel, towelette, or other material in order to obtain a sample of bacteria from the surface. Once the sample has been taken, the surface of the material may be tested in order to determine if a component is present.

Depending on the component to be tested, a resulting recommended treatment may be suggested. For example, if the presence of ammonia oxidizing bacteria is determined, then no action may be taken. Alternatively, additional ammonia oxidizing bacteria, ammonia, ammonium ions, or urea may be administered or applied at a pre-determined concentration or amount to the subject, *e.g*., a surface of the subject, *e.g*., the skin. This may depend on whether the presence or level detected is determined as adequate, *e.g*., in a range of values that would be appropriate for treatment of a condition of a subject.

If presence of ammonia, ammonium ions, or urea is determined, then an action may or may not be taken. For example, if presence of ammonia, ammonium ions, or urea is determined, at least one of ammonia oxidizing bacteria, ammonia, ammonium ions, and urea, may or may not be administered or applied at a pre-determined concentration or amount to the subject, *e.g*., a surface of the subject, *e.g*., the skin..

If the presence of one or more pathogenic bacteria is determined, then an action may or may not be taken. For example, if presence of pathogenic bacteria is determined, at least one of ammonia oxidizing bacteria, ammonia, ammonium ions, and urea, may or may not be administered or applied at a pre-determined concentration or amount to the subject, *e.g*., a surface of the subject, *e.g*., the skin.

If the presence of one or more of nitrite and nitric oxide is determined, then an action may or may not be taken. For example, if presence of nitrite and/or nitric oxide is determined, at least one of ammonia oxidizing bacteria, ammonia, ammonium ions, and urea, may or may not be administered or applied at a pre-determined concentration or amount to the subject, *e.g*., a surface of the subject, *e.g*., the skin.

A level of a component that is present may be determined. This may be a quantitative or qualitative determination. For example, an amount or concentration of a component may be determined. This amount or concentration may be used to determine whether administration or application of ammonia oxidizing bacteria is required or desired, or whether administration or application of other compounds, *e.g*., ammonia, ammonium ions or urea is required or desired.

The determined amount or concentration may be compared to a range of values corresponding to a pre-determined range of amount or concentration. The amount or concentration may be determined to be within or outside of the range of values corresponding to the pre-determined range.

In certain aspects, if the amount or concentration is in the range of values corresponding to the pre-determined range, the product may be classified as accepted. In certain other aspects, if the amount or concentration is outside the range of values corresponding to the pre-determined range, the product may be classified as accepted.

In certain aspects, if the amount or concentration is in the range of values corresponding to the pre-determined range, the product may be classified as not accepted. In certain other aspects, if the amount or concentration is outside the range of values corresponding to the pre-determined range, the product may be classified as not accepted.

If the amount or concentration is classified as not accepted, administration or application of at least one of ammonia oxidizing bacteria, ammonia, ammonium ions, and urea may be performed. If the amount or concentration is classified as accepted, no administration or application of at least one of ammonia oxidizing bacteria, ammonia, ammonium ions, and urea may be performed.

The pre-determined range of concentrations of ammonia oxidizing bacteria may be between about 10⁸ CFU/L and about 10¹⁴ CFU/L. In certain aspects, the pre-determined range of concentrations of ammonia oxidizing bacteria is less than about 10⁸ or between about 10⁸ - 10⁹, 10⁹ - 10¹⁰, 10¹⁰ - 10¹¹, 10¹¹ - 10¹², 10¹² - 10¹³, or 10¹³ - 10¹⁴ CFU/L.

The pre-determined range of concentrations of ammonia oxidizing bacteria may be between about 10⁸ CFU/ml and about 10¹⁴ CFU/ml. In certain aspects, the pre-determined range of concentrations of ammonia oxidizing bacteria is less than about 10⁸ or between about 10⁸ - 10⁹, 10⁹ - 10¹⁰, 10¹⁰ - 10¹¹, 10¹¹ - 10¹², 10¹² - 10¹³, or 10¹³ - 10¹⁴ CFU/ml.

The pre-determined range of amounts of ammonia oxidizing bacteria may comprise between about 0.1 milligrams (mg) and about 1000 mg of ammonia oxidizing bacteria. In certain aspects, the pre-determined range may be between about 50 mg and about 1000 mg of ammonia oxidizing bacteria. The range may comprise between about 0.1-0.5 mg, 0.2-0.7 mg, 0.5-1.0 mg, 0.5-2 mg, 0.5-5 mg, 2.5-5 mg, 2.5-7.0 mg, 5.0-10 mg, 7.5-15 mg, 10-15 mg, 15-20 mg, 15-25 mg, 20-30 mg, 25-50 mg, 25-75 mg, 50-75 mg, 50-100 mg, 75-100 mg, 100-200 mg, 200-300 mg, 300-400 mg, 400-500 mg, 500-600 mg, 600-700 mg, 700-800 mg, 800-900 mg, 900-1000 mg, 100-250 mg, 250-500 mg, 100-500 mg, 500-750 mg, 750-1000 mg, or 500-1000 mg.

The concentration of ammonia oxidizing bacteria administered or applied may be between about 10⁸ CFU/L to about 10¹⁴ CFU/L. In certain aspects, the concentration of ammonia oxidizing bacteria administered or applied may be less than about 10⁸ or between about 10⁸ - 10⁹, 10⁹ - 10¹⁰, 10¹⁰ - 10¹¹, 10¹¹ - 10¹², 10¹² - 10¹³, or 10¹³ - 10¹⁴ CFU/L.

The concentration of ammonia oxidizing bacteria administered or applied may be between about 10⁸ CFU/ml to about 10¹⁴ CFU/ml. In certain aspects, the concentration of ammonia oxidizing bacteria administered or applied may be less than about 10⁸ or between about 10⁸ - 10⁹, 10⁹ - 10¹⁰, 10¹⁰ - 10¹¹, 10¹¹ - 10¹², 10¹² - 10¹³, or 10¹³ - 10¹⁴ CFU/ml.

The amount of ammonia oxidizing bacteria administered or applied may comprise between about 0.1 milligrams (mg) and about 1000 mg of ammonia oxidizing bacteria. In certain aspects, the amount administered or applied may comprise between about 50 mg and about 1000 mg of ammonia oxidizing bacteria. The amount administered or applied may comprise between about 0.1-0.5 mg, 0.2-0.7 mg, 0.5-1.0 mg, 0.5-2 mg, 0.5-5 mg, 2.5-5 mg, 2.5-7.0 mg, 5.0-10 mg, 7.5-15 mg, 10-15 mg, 15-20 mg, 15-25 mg, 20-30 mg, 25-50 mg, 25-75 mg, 50-75 mg, 50-100 mg, 75-100 mg, 100-200 mg, 200-300 mg, 300-400 mg, 400-500 mg, 500-600 mg, 600-700 mg, 700-800 mg, 800-900 mg, 900-1000 mg, 100-250 mg, 250-500 mg, 100-500 mg, 500-750 mg, 750-1000 mg, or 500-1000 mg.

### 6. Methods of Preparing AOB Product

Methods of preparing a product comprising ammonia oxidizing bacteria are disclosed herein. The preparation may or may not involve adding additional, *e.g*., exogenous ammonia oxidizing bacteria to provide an ammonia oxidizing bacteria-containing product, or supplementing a product with additional ammonia oxidizing bacteria.

This method may comprise measuring an amount or a concentration of ammonia oxidizing bacteria in a preparation. The measurement may provide a value. Responsive to that value, the preparation may be classified as requiring ammonia oxidizing bacteria or not requiring ammonia oxidizing bacteria.

The method may further comprise, if the preparation is classified as requiring ammonia oxidizing bacteria, adding an amount or concentration of ammonia oxidizing bacteria. In addition, or in the alternative, the method may further comprise adding an amount or concentration of at least one of ammonia, ammonium ions, and urea to the preparation. This may be based on the value that was measured. The addition of one or more of these components may provide a product that may be released into commerce.

In certain aspects, responsive to the value measure, the preparation may be passed into a next step of releasing into commerce the product, without addition of ammonia oxidizing bacteria.

The product (including preparations) provided by the methods discussed herein may be packaged into a package, *e.g*., a container, which may then be placed in commerce. The packaging may comprise an aseptic or sterile compartment. The packaging may be substantially free of other organisms. The packaging may be substantially preservative-free or contain preservative or other components, *e.g*., excipients or chelator as discussed in this disclosure.

The products may be provided in a container, delivery system, or delivery device. The ammonia oxidizing bacteria may be in a growth state or a storage state. Other components, such as ammonia, ammonium ions, and/or urea may be included in the container. The additional components may be in a separate compartment or chamber as the ammonia oxidizing bacteria, and upon actuation of the container, delivery system, or delivery device, or upon delivery of the contents, the ammonia oxidizing bacteria may contact, *e.g*., be mixed, with the other components of the container.

### 7. Fortifying Natural Products

Natural products may be fortified by the present disclosure. Natural product or fortified natural product may comprise at least one of mud, water, food-derived products, plant-derived products, extracts, and oils. The natural products may be used in cosmetic and therapeutic applications, *e.g*., in cosmetic products, *e.g*., therapeutic products, *e.g*., spa treatments.

The natural product or the fortified natural product may be incorporated into at least one of a powder, cream, lotion, wrap, scrub, eye mask, facial mask, body mask, aerosol, spray, salve, wipe, stick, bandage, or soak.

The natural product or fortified natural product may be provided as, or is disposed in at least one of a baby product, *e.g*., a baby shampoo, a baby lotion, a baby oil, a baby powder, a baby cream; a bath preparation, *e.g*., a bath oil, a tablet, a salt, a bubble bath, a bath capsule; an eye makeup preparation, *e.g*., an eyebrow pencil, an eyeliner, an eye shadow, an eye lotion, an eye makeup remover, a mascara; a fragrance preparation, *e.g*., a colognes, a toilet water, a perfume, a powder (dusting and talcum), a sachet; hair preparations, *e.g.*, hair conditioners, hair sprays, hair straighteners, permanent waves, rinses, shampoos, tonics, dressings, hair grooming aids, wave sets; hair coloring preparations, *e.g*., hair dyes and colors, hair tints, coloring hair rinses, coloring hair shampoos, hair lighteners with color, hair bleaches; makeup preparations, *e.g*., face powders, foundations, leg and body paints, lipstick, makeup bases, rouges, makeup fixatives; manicuring preparations, *e.g*., basecoats and undercoats, cuticle softeners, nail creams and lotions, nail extenders, nail polish and enamel, nail polish and enamel removers; oral hygiene products, *e.g*., dentrifices, mouthwashes and breath fresheners; bath soaps and detergents, deodorants, douches, feminine hygiene deodorants; shaving preparations, *e.g.,* aftershave lotions, beard softeners, talcum, preshave lotions, shaving cream, shaving soap; skin care preparations, *e.g*., cleansing, depilatories, face and neck, body and hand, foot powders and sprays, moisturizing, night preparations, paste masks, skin fresheners; and suntan preparations, *e.g*., gels, creams, and liquids, and indoor tanning preparations.

The natural products may be fortified with ammonia oxidizing bacteria. In addition, or in the alternative, the natural product may be fortified with ammonia, ammonium ions, and/or urea. In certain aspects, the natural products may naturally comprise ammonia oxidizing bacteria (that is, obtained from natural sources without the addition of ammonia oxidizing bacteria, *e.g*., without human intervention). In certain aspects, the natural products may naturally comprise ammonia, ammonium ions, and/or urea. The ammonia oxidizing bacterial may be in a growth state or a storage state.

Fortifying natural products may comprise measuring an amount or a concentration of ammonia oxidizing bacteria in the natural product. The measurement may provide a value. Responsive to that value, the natural product may be classified as requiring ammonia oxidizing bacteria, or classifying the natural product as not requiring ammonia oxidizing bacteria.

An amount or a concentration of ammonia oxidizing bacteria may be added to the natural product. This may be performed responsive to the value measured, and/or in response to the classification as described above. This may provide a fortified natural product.

An amount or a concentration of ammonia, ammonium ions, and urea may be added to the natural product. This may be performed responsive to the value measured, and/or in response to the classification as described above. This may provide a fortified natural product.

In response to the value measured, the natural product may be passed to the next step of releasing the natural product into commerce without addition of ammonia oxidizing bacteria.

A fortified natural product may also be passed to the next step of releasing the fortified natural product into commerce.

The product, *e.g*., the natural product or the fortified natural product (including preparations comprising one or more natural products) provided by the methods discussed herein may be packaged into a package. *e.g*., a container, which may then be placed in commerce. The packaging may comprise an aseptic or sterile compartment. The package may be substantially free of other organisms. The packaging may be substantially preservative-free or contain preservative or other components, *e.g*., excipients or chelator as discussed in this disclosure.

The products may be provided in a container, delivery system, or delivery device. The ammonia oxidizing bacteria may be in a growth state or a storage state. Other components, such as ammonia, ammonium ions, and/or urea may be included in the container. The additional components may be in a separate compartment or chamber as the ammonia oxidizing bacteria, and upon actuation of the container, delivery system, or delivery device, or upon delivery of the contents, the ammonia oxidizing bacteria may contact, *e.g*., be mixed, with the other components of the container.

The weight of the package, container, delivery system, or delivery device, with or without contents may be is less than about 50, 100, 200, 300, 400, 500, 600, 700, 800, 900, 1000, 1500, or 2000 grams.

The package, natural product or the fortified natural product may be provided as, disposed in, or comprise a powder, cosmetic, cream, stick, aerosol, salve, wipe, or bandage.

The range of concentrations of ammonia oxidizing bacteria that the natural product may be fortified with or may comprise may be between about 10⁸ CFU/L to about 10¹⁴ CFU/L. In certain aspects, the range of concentrations of ammonia oxidizing bacteria is less than about 10⁸ or between about 10⁸ - 10⁹, 10⁹ - 10¹⁰, 10¹⁰ - 10¹¹, 10¹¹ - 10¹², 10¹² - 10¹³, or 10¹³ - 10¹⁴ CFU/L.

The range of concentrations of ammonia oxidizing bacteria that the natural product may be fortified with or may comprise may be between about 10⁸ CFU/ml to about 10¹⁴ CFU/ml. In certain aspects, the range of concentrations of ammonia oxidizing bacteria is less than about 10⁸ or between about 10⁸ - 10⁹, 10⁹ - 10¹⁰, 10¹⁰ - 10¹¹, 10¹¹ - 10¹², 10¹² - 10¹³, or 10¹³ - 10¹⁴ CFU/ml.

The range of amounts of ammonia oxidizing bacteria that the natural product may be fortified with or may comprise may be between about 0.1 milligrams (mg) and about 1000 mg of ammonia oxidizing bacteria. In certain aspects, the range may be between about 50 mg and about 1000 mg of ammonia oxidizing bacteria. The range may comprise between about 0.1-0.5 mg, 0.2-0.7 mg, 0.5-1.0 mg, 0.5-2 mg, 0.5-5 mg, 2.5-5 mg, 2.5-7.0 mg, 5.0-10 mg, 7.5-15 mg, 10-15 mg, 15-20 mg, 15-25 mg, 20-30 mg, 25-50 mg, 25-75 mg, 50-75 mg, 50-100 mg, 75-100 mg, 100-200 mg, 200-300 mg, 300-400 mg, 400-500 mg, 500-600 mg, 600-700 mg, 700-800 mg, 800-900 mg, 900-1000 mg, 100-250 mg, 250-500 mg, 100-500 mg, 500-750 mg, 750-1000 mg, or 500-1000 mg.

The natural product or fortified natural product may comprise a mass ratio of ammonia oxidizing bacteria to an excipient, *e.g*., a pharmaceutically acceptable excipient or a cosmetically acceptable excipient in a range of about 0.1 grams per liter to about 1 gram per liter. The natural product or fortified natural product may comprise a mass ratio of ammonia oxidizing bacteria to an excipent in a range of about 0.1-0.2, 0.2-0.3, 0.1-0.5, 0.2-0.7, 0.5-1.0, or 0.7-1.0 grams per liter.

### 8. Compositions comprising ammonia oxidizing bacteria

The present disclosure provides, *inter alia,* compositions comprising ammonia oxidizing bacteria, *e.g.*, a preparation of ammonia oxidizing bacteria, or a purified preparation of ammonia oxidizing bacteria, *e.g*., a natural product, or a fortified natural product. The compositions comprising ammonia oxidizing bacteria, *e.g*., a preparation of ammonia oxidizing bacteria, or a purified preparation of ammonia oxidizing bacteria *e.g*., a natural product, or a fortified natural product may be provided in a cosmetic product or a therapeutic product. The preparation may comprise, *inter alia*, at least one of ammonia, ammonium ions, and urea.

In some aspects, the present disclosure provides compositions with a defined number of species. For instance, this disclosure provides a composition having ammonia oxidizing bacteria, or more specifically having one genus of ammonia oxidizing bacteria, or more specifically, having one species of ammonia oxidizing *e.g*., *N. eutropha*, and one other type of organism, and no other types of organism. In other examples, the composition has ammonia oxidizing bacteria, or more specifically has one genus of ammonia oxidizing bacteria, or more specifically, having one species of ammonia oxidizing *e.g*., *N. eutropha* and 2, 3, 4, 5, 6, 7, 8, 9, or 10 other types of organism, and no other types of organism. Suitable ammonia-oxidizing bacteria for this purpose include those in the genera *Nitrosomonas*, *Nitrosococcus*, *Nitrosospira*, *Nitrosocystis*, *Nitrosolobus*, or *Nitrosovibrio.*

In some instances, one or more other organisms besides ammonia oxidizing bacteria may be included in the preparation of ammonia oxidizing bacteria. For example, an organism of the genus selected from the group consisting of *Lactobacillus*, *Streptococcus*, *Bifidobacter*, and combinations thereof, may be provided in the preparation of ammonia oxidizing bacteria. In some instances, the preparation may be substantially free of other organisms.

In some instances, the composition comprising ammonia oxidizing bacteria provides conditions that support ammonia oxidizing bacteria viability. For instance, the composition may promote ammonia oxidizing bacteria growth and metabolism or may promote a dormant state (*e.g*., freezing) or storage state as described herein from which viable ammonia oxidizing bacteria can be recovered. When the composition promotes growth or metabolism, it may contain water and/or nutrients that ammonia oxidizing bacteria consumes, *e.g*., as ammonium ions, ammonia, urea, oxygen, carbon dioxide, or trace minerals.

Preparations of ammonia oxidizing bacteria may comprise between about between about 10⁸ to about 10¹⁴ CFU/L. The preparation may comprise at least about 10⁸, 10⁹, 10¹⁰, 10¹¹, 2 x 10¹¹, 5 x 10¹¹, 10¹², 2 x 10¹², 5 x 10¹², 10¹³, 2 x 10¹³, 5 x 10¹³, or 10¹⁴; or about 10⁸-10⁹, 10⁹-10¹⁰, 10¹⁰-10¹¹, 10¹¹-10¹², 10¹²-10¹³, or 10¹³-10¹⁴ CFU/L.

Preparations of ammonia oxidizing bacteria may comprise between about between about 10⁸ to about 10¹⁴ CFU/ml. The preparation may comprise at least about 10⁸, 10⁹, 10¹⁰, 10¹¹, 2 x 10¹¹, 5 x 10¹¹, 10¹², 2 x 10¹², 5 x 10¹², 10¹³, 2 x 10¹³, 5 x 10¹³, or 10¹⁴; or about 10⁸-10⁹, 10⁹-10¹⁰, 10¹⁰-10¹¹, 10¹¹-10¹², 10¹²-10¹³, or 10¹³-10¹⁴ CFU/ml.

In some instances, the preparation may comprise between about 1 x 10⁹ to about 10 x 10⁹ CFU/L. In some instances, the preparation may comprise about 3 x 10¹⁰ CFU, *e.g.*, 3 x 10¹⁰ CFU per day. In some instances, the preparation may comprise about 1 x 10⁹ to about 10 x 10⁹ CFU, *e.g*., about 1 x 10⁹ to about 10 x 10⁹ CFU per day.

In some instances, the preparation of ammonia oxidizing bacteria may comprise between about 0.1 milligrams (mg) and about 1000 mg of ammonia oxidizing bacteria. In certain aspects, the preparation may comprise between about 50 mg and about 1000 mg of ammonia oxidizing bacteria. The preparation may comprise between about 0.1-0.5 mg, 0.2-0.7 mg, 0.5-1.0 mg, 0.5-2 mg, 0.5-5 mg, 2.5-5 mg, 2.5-7.0 mg, 5.0-10 mg, 7.5-15 mg, 10-15 mg, 15-20 mg, 15-25 mg, 20-30 mg, 25-50 mg, 25-75 mg, 50-75 mg, 50-100 mg, 75-100 mg, 100-200 mg, 200-300 mg, 300-400 mg, 400-500 mg, 500-600 mg, 600-700 mg, 700-800 mg, 800-900 mg, 900-1000 mg, 100-250 mg, 250-500 mg, 100-500 mg, 500-750 mg, 750-1000 mg, or 500-1000 mg.

In some instances, the preparation of ammonia oxidizing bacteria my comprise a mass ratio of ammonia oxidizing bacteria to an excipient, *e.g.,* a pharmaceutically acceptable excipient or a cosmetically acceptable excipient in a range of about 0.1 grams per liter to about 1 gram per liter. The preparation may comprise a mass ratio of ammonia oxidizing bacteria to an excipent in a range of about 0.1-0.2, 0.2-0.3, 0.1-0.5, 0.2-0.7, 0.5-1.0, or 0.7-1.0 grams per liter.

In some instances, the preparation of ammonia oxidizing bacteria may be ammonia oxidizing bacteria in a buffer solution comprising, consisting essentially of, or consisting of disodium phosphate and magnesium chloride, for example, 50 mM Na₂HPO₄ and 2 mM MgCl₂. The preparation may be provided in a buffer at a pre-determined volume of, for example, between about 0.1 and about 100 fluid ounces, about 0.2 and about 50 fluid ounces, about 0.5 and about 25 fluid ounces, about 1.0 and about 10 fluid ounces, about 2.0 and about 7 fluid ounces, about 3 and about 5 fluid ounces. In some instances, the preparation may be provided in a container. The preparation may be provided in a container constructed to contain about 3.4 fluid ounces, or any other volume disclosed herein. The preparation may be in a form that may be capable of being aerosolized, sprayed or misted, i.e., in the form of a mist.

The ammonia oxidizing bacteria may be combined with one or more excipients, *e.g*., one or more pharmaceutically acceptable excipients or cosmetically acceptable excipients. In some instances, "pharmaceutically acceptable excipient" refers to a pharmaceutically-acceptable material, composition, or vehicle, such as a liquid or solid filler, diluent, solvent, or encapsulating material. In some instances, each excipient is "pharmaceutically acceptable" in the sense of being compatible with the other ingredients of a pharmaceutical formulation, and suitable for use in contact with the tissue or organ of humans and animals without excessive toxicity, irritation, allergic response, immunogenicity, or other problems or complications, commensurate with a reasonable benefit/risk ratio. See, Remington: The Science and Practice of Pharmacy, 21st ed.; Lippincott Williams & Wilkins: Philadelphia, Pa., 2005; Handbook of Pharmaceutical Excipients, 6th ed.; Rowe et al., Eds.; The Pharmaceutical Press and the American Pharmaceutical Association: 2009; Handbook of Pharmaceutical Additives, 3rd ed.; Ash and Ash Eds.; Gower Publishing Company: 2007; Pharmaceutical Preformulation and Formulation, 2nd ed.; Gibson Ed.; CRC Press LLC: Boca Raton, Fla., 2009.

In some instances, a cosmetically acceptable excipient refers to a cosmetically acceptable material, composition, or vehicle, such as a liquid or solid filler, diluent, solvent, or encapsulating material. In some instances, each excipient is cosmetically acceptable in the sense of being compatible with the other ingredients of a cosmetic formulation, and suitable for use in contact with the tissue or organ of humans and animals without excessive toxicity, irritation, allergic response, immunogenicity, or other problems or complications, commensurate with a reasonable benefit/risk ratio.

While it is possible for the active ingredient, *e.g*., ammonia oxidizing bacteria, to be administered alone, in many instances it is present in a pharmaceutical formulation, preparation, or composition, or a cosmetic formulation, preparation, or composition. Accordingly, this disclosure provides a pharmaceutical formulation (preparation or composition) or a cosmetic formulation (preparation or composition) comprising ammonia oxidizing bacteria and a pharmaceutically acceptable excipient or a cosmetically acceptable excipient. Pharmaceutical compositions and cosmetic compositions may take the form of a formulation as described below.

The pharmaceutical and cosmetic formulations (*e.g*., preparations or compositions) described herein may include those suitable for oral (*e.g*., by way of, or for the purposes of depositing in the gastrointestinal tract), parenteral (including subcutaneous, intradermal, intramuscular, intravenous, and intraarticular), inhalation (including fine particle dusts or mists which may be generated by means of various types of metered doses, pressurized aerosols, nebulizers or insufflators, and including intranasally (nasal) or via the lungs (pulmonary)), rectal and topical (including dermal, transdermal, transmucosal, buccal, sublingual, and intraocular) administration, although the most suitable route may depend upon, for example, the condition and disorder of the recipient.

The formulations (*e.g*., preparations or compositions) may conveniently be presented in unit dosage form and may be prepared by any of the methods known in the art of pharmacy or cosmetology. Typically, methods include the step of bringing the active ingredient (*e.g*., ammonia oxidizing bacteria) into association with a pharmaceutical or a comestic carrier which constitutes one or more accessory ingredients. In general, the formulations are prepared by uniformly and intimately bringing into association the active ingredient with liquid carriers or finely divided solid carriers or both and then, if necessary, shaping the product into the desired formulation.

Formulations may be presented as discrete units such as capsules, cachets or tablets, each containing a predetermined amount of ammonia oxidizing bacteria; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste. Various pharmaceutically acceptable carriers and their formulation are described in standard formulation treatises, *e.g*., Remington's Pharmaceutical Sciences by E. W. Martin. See also Wang, Y. J. and Hanson, M. A., Journal of Parenteral Science and Technology, Technical Report No. 10, Supp. 42:2 S, 1988.

The ammonia oxidizing bacteria formulations, compositions, or preparations, can, for example, be administered in a form suitable for immediate release or controlled (extended) release. Suitable examples of sustained-release systems include suitable polymeric materials, for example semi-permeable polymer matrices in the form of shaped articles, *e.g*., films, or microcapsules; suitable hydrophobic materials, for example as an emulsion in an acceptable oil; or ion exchange resins. Controlled (sustained)-release systems may be administered orally; rectally; parenterally; intracistemally; intravaginally; intraperitoneally; topically, for example as a powder, ointment, gel, drop or transdermal patch; bucally; or as a spray.

Preparations for administration can be suitably formulated to give controlled release of ammonia oxidizing bacteria. For example, the formulations, preparations, or compositions may be in the form of particles comprising one or more of biodegradable polymers, polysaccharide jellifying and/or bioadhesive polymers, or amphiphilic polymers. These compositions exhibit certain biocompatibility features which allow a controlled release of an active substance. See U.S. Pat. No. 5,700,486. The preparation may comprise a controlled release material.

Exemplary compositions, *e.g*., as a preparation, may include suspensions which can contain, for example, microcrystalline cellulose for imparting bulk, alginic acid or sodium alginate as a suspending agent, methylcellulose as a viscosity enhancer, dicalcium phosphate, starch, magnesium stearate and/or lactose and/or other excipients, binders, extenders, disintegrants, diluents and lubricants, mannitol, lactose, sucrose and/or cyclodextrins. Also included in such formulations may be high molecular weight excipients such as celluloses (avicel) or polyethylene glycols (PEG). Such formulations can also include an excipient to aid mucosal adhesion such as hydroxy propyl cellulose (HPC), hydroxy propyl methyl cellulose (HPMC), sodium carboxy methyl cellulose (SCMC), maleic anhydride copolymer (*e.g*., Gantrez), and agents to control release such as polyacrylic copolymer (*e.g.* Carbopol 934). Lubricants, surfactants, glidants, flavors, coloring agents and stabilizers may also be added for ease of fabrication and use. The surfactant may be a zwitterionic surfactant, a nonionic surfactant, or an anionic surfactant.

Excipients, such as surfactants that may be used with instances of the present disclosure may include one or more of cocamidopropyl betaine (ColaTeric COAB), polyethylene sorbitol ester (*e.g*., Tween 80), ethoxylated lauryl alcohol (RhodaSurf 6 NAT), sodium laureth sulfate/lauryl glucoside/cocamidopropyl betaine (Plantapon 611 L UP), sodium laureth sulfate (*e.g*., RhodaPex ESB 70 NAT), alkyl polyglucoside (*e.g*., Plantaren 2000 N UP), sodium laureth sulfate (Plantaren 200), Dr. Bronner's Castile soap, Dr. Bronner's Castile baby soap, Lauramine oxide (ColaLux Lo), sodium dodecyl sulfate (SDS), polysulfonate alkyl polyglucoside (PolySufanate 160 P), sodium lauryl sulfate (Stepanol-WA Extra K). and combinations thereof. Dr. Bronner's Castile soap comprises water, organic coconut oil, potassium hydroxide, organic olive oil, organic fair deal hemp oil, organic jojoba oil, citric acid, and tocopherol.

In some instances, surfactants may be used with ammonia oxidizing bacteria in amounts that allow nitrite production to occur. In some instances, the preparation may have less than about 0.0001 % to about 10% of surfactant. In some instances, the preparation may have between about 0.1 % and about 10 % surfactant. In some instances, the concentration of surfactant used may be between about 0.0001 % and about 10%. In some instances, the preparation may be substantially free of surfactant.

In some instances, the formulation, *e.g*., preparation, may include other components that may enhance effectiveness of ammonia oxidizing bacteria, or enhance a treatment or indication.

In some instances, a chelator may be included in the preparation. A chelator may be a compound that may bind with another compound, *e.g*., a metal. The chelator may provide assistance in removing an unwanted compound from an environment, or may act in a protective manner to reduce or eliminate contact of a particular compound with an environment, *e.g*., ammonia oxidizing bacteria, *e.g.* a preparation of ammonia oxidizing bacteria, *e.g*., an excipient. In some instances, the preparation may be substantially free of chelator.

Formulations (*e.g*., preparations, including natural products and fortified natural products) may also contain anti-oxidants, buffers, bacteriostats that prevent the growth of undesired bacteria, solutes, and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of a sterile liquid carrier, for example saline or water-for-injection, immediately prior to use. Extemporaneous solutions and suspensions may be prepared from powders, granules and tablets of the kind previously described. Exemplary compositions include solutions or suspensions which can contain, for example, suitable non-toxic, pharmaceutically acceptable diluents or solvents, such as mannitol, 1,3-butanediol, water, Ringer's solution, an isotonic sodium chloride solution, or other suitable dispersing or wetting and suspending agents, including synthetic mono- or diglycerides, and fatty acids, including oleic acid, or Cremaphor. An aqueous carrier may be, for example, an isotonic buffer solution at a pH of from about 3.0 to about 8.0, a pH of from about 3.5 to about 7.4, for example from 3.5 to 6.0, for example from 3.5 to about 5.0. Useful buffers include sodium citrate-citric acid and sodium phosphate-phosphoric acid, and sodium acetate/acetic acid buffers. The composition in some instances does not include oxidizing agents.

Excipients that can be included are, for instance, proteins, such as human serum albumin or plasma preparations. If desired, the pharmaceutical composition, *e.g*., a preparation, may also contain minor amounts of non-toxic auxiliary substances, such as wetting or emulsifying agents, surfactants, preservatives, and pH buffering agents and the like, for example sodium acetate or sorbitan monolaurate. In some instances, excipients, *e.g*., a pharmaceutically acceptable excipient or a cosmetically acceptable excipient, may comprise an anti-adherent, binder, coat, disintegrant, filler, flavor, color, lubricant, glidant, sorbent, preservative, or sweetener. In some instances, the preparation may be substantially free of excipients.

In some instances, the preparation may be substantially free of one or more of the compounds or substances listed in the disclosure.

Exemplary compositions for aerosol administration include solutions in saline, which can contain, for example, benzyl alcohol or other suitable preservatives, absorption promoters to enhance bioavailability, and/or other solubilizing or dispersing agents. Conveniently in compositions for aerosol administration the ammonia oxidizing bacteria may be delivered in the form of an aerosol spray presentation from a pressurized pack or a nebulizer, with the use of a suitable propellant, *e.g*., dichlorodifluoro-methane, trichlorofluoromethane, dichlorotetrafluoroethane, carbon dioxide or other suitable gas. In the case of a pressurized aerosol the dosage unit can be determined by providing a valve to deliver a metered amount. Capsules and cartridges of *e.g*., gelatin can be formulated to contain a powder mix of the ammonia oxidizing bacteria and a suitable powder base, for example lactose or starch. In certain instances, ammonia oxidizing bacteria is administered as an aerosol from a metered dose valve, through an aerosol adapter also known as an actuator. Optionally, a stabilizer is also included, and/or porous particles for deep lung delivery are included (*e.g*., see U.S. Pat. No. 6,447,743). The composition or preparation may be in a form that may be capable of being aerosolized, sprayed or misted, i.e., in the form of a mist. The preparation of ammonia oxidizing bacteria may be ammonia oxidizing bacteria in a buffer solution comprising, consisting essentially of, or consisting of disodium phosphate and magnesium chloride, for example, 50 mM Na₂HPO₄ and 2 mM MgCl₂.

Formulations may be presented with carriers such as cocoa butter, synthetic glyceride esters or polyethylene glycol. Such carriers are typically solid at ordinary temperatures, but liquefy and/or dissolve at body temperature to release the ammonia oxidizing bacteria.

Exemplary compositions for topical administration include a topical carrier such as Plastibase (mineral oil gelled with polyethylene). In some aspects, the composition, *e.g*., preparation, and/or excipient may be in the form of one or more of a liquid, a solid, or a gel. For example, liquid suspensions may include, but are not limited to, water, saline, phosphate-buffered saline, or an ammonia oxidizing storage buffer.

Gel formulations may include, but are not limited to agar, silica, polyacrylic acid (for example Carbopol®), carboxymethul cellulose, starch, guar gum, alginate or chitosan.

In some instances, the formulation, *e.g*., preparation, may be supplemented with an ammonia source including, but not limited to one or more of ammonia, ammonium ions, *e.g*., ammonium chloride or ammonium sulfate, and urea.

In some instances, an ammonia oxidizing bacteria composition, *e.g*., preparation, is formulated to improve NO penetration into the skin. A gel-forming material such as KY jelly or various hair gels would present a diffusion barrier to NO loss to ambient air, and so improve the skin's absorption of NO. The NO level in the skin will generally not greatly exceed 20 nM/L because that level activates GC and would cause local vasodilatation and oxidative destruction of excess NO.

It should be understood that in addition to the ingredients particularly mentioned above, the formulations, *e.g*., preparations, as described herein may include other agents conventional in the art having regard to the type of formulation in question.

The formulation, *e.g*., preparation, *e.g*., composition may be provided in a container, delivery system, or delivery device, having a weight, including or not including the contents of the container, that may be less than about 50, 100, 200, 300, 400, 500, 600, 700, 800, 900, 1000, 1500, or 2000 grams.

Suitable unit dosage formulations are those containing an effective dose, as hereinbefore recited, or an appropriate fraction thereof, of ammonia oxidizing bacteria.

A therapeutically effective amount of ammonia oxidizing bacteria may be administered as a single pulse dose, as a bolus dose, or as pulse doses administered over time. Thus, in pulse doses, a bolus administration of ammonia oxidizing bacteria is provided, followed by a time period wherein ammonia oxidizing bacteria is administered to the subject, followed by a second bolus administration. In specific, non-limiting examples, pulse doses are administered during the course of a day, during the course of a week, or during the course of a month.

In some instances, a preparation of ammonia oxidizing bacteria, *e.g*., a formulation, *e.g*., a composition, may be applied for a pre-determined number of days. This may be based, for example, at least in part, on the severity of the condition or disease, the response to the treatment, the dosage applied and the frequency of the dose. For example, the preparation may be applied for about 1-3, 3-5, 5-7, 7-9, 5-10, 10-14, 12-18, 12-21, 21-28, 28-35, 35-42, 42-49, 49-56, 46-63, 63-70, 70-77, 77-84, 84-91 days, *e.g*., for about 1 month, for about 2 months, for about 3 months. In some instances, the ammonia oxidizing bacteria is administered for an indefinite period of time, e.g, greater than one year, greater than 5 years, greater than 10 years, greater than 15 years, greater than 30 years, greater than 50 years, greater than 75 years.

In some instances, a preparation of ammonia oxidizing bacteria, *e.g*., a formulation, *e.g*., a composition, may be applied a pre-determined number of times per day. This may be based, for example, at least in part, on the severity of the condition or disease, the response to the treatment, the dosage applied and the frequency of the dose. For example, the preparation may be applied about 1, 2, 3, 4, 5, 6, 7, 8, 9, 10, 11, 12, 13, 14, 15, 16, 17, 18, 19, 20, 21, 22, 23, 24 times per day.

In some instances, the preparation may be applied one time per day. In other instances, the preparation may be applied two times per day. In some instances, the preparation may be applied a first pre-determined amount for a certain number of days, and a second pre-determined amount for a certain subsequent number of days. In some instances, the preparation may be applied for about 16 days.

Ammonia oxidizing bacteria may be associated with a variety of consumer products, and examples of such products are set out below. In some instances, the ammonia oxidizing bacteria associated with a product is admixed with the product, for example, spread evenly throughout the product, and in some instances, the ammonia oxidizing bacteria associated with a product is layered on the product.

In some instances, the ammonia oxidizing bacteria is associated with a powder. Powders are typically small particulate solids that are not attached to each other and that can flow freely when tilted. Exemplary powders for consumer use include talcum powder and some cosmetics (e.g., powder foundation, including pressed powders). Other powders may be contemplated for use in conjunction with ammonia oxidizing bacteria systems and methods of the present disclosure.

In some instances, the ammonia oxidizing bacteria is associated with a cosmetic. The cosmetic may be a substance for topical application intended to alter a person's appearance, *e.g.,* a liquid foundation, a powder foundation, blush, or lipstick. The cosmetic may be any substance recited in the Food and Drug Administration regulations, e.g., under 21 C.F.R.§ 720.4.

The preparation, *e.g.,* cosmetic may be at least one of a baby product, *e.g*., a baby shampoo, a baby lotion, a baby oil, a baby powder, a baby cream; a bath preparation, *e.g*., a bath oil, a tablet, a salt, a bubble bath, a bath capsule; an eye makeup preparation, *e.g*., an eyebrow pencil, an eyeliner, an eye shadow, an eye lotion, an eye makeup remover, a mascara; a fragrance preparation, *e.g*., a colognes, a toilet water, a perfume, a powder (dusting and talcum), a sachet; hair preparations, *e.g*., hair conditioners, hair sprays, hair straighteners, permanent waves, rinses, shampoos, tonics, dressings, hair grooming aids, wave sets; hair coloring preparations, *e.g*., hair dyes and colors, hair tints, coloring hair rinses, coloring hair shampoos, hair lighteners with color, hair bleaches; makeup preparations, *e.g*., face powders, foundations, leg and body paints, lipstick, makeup bases, rouges, makeup fixatives; manicuring preparations, *e.g*., basecoats and undercoats, cuticle softeners, nail creams and lotions, nail extenders, nail polish and enamel, nail polish and enamel removers; oral hygiene products, *e.g*., dentrifices, mouthwashes and breath fresheners; bath soaps and detergents, deodorants, douches, feminine hygiene deodorants; shaving preparations, *e.g*., aftershave lotions, beard softeners, talcum, preshave lotions, shaving cream, shaving soap; skin care preparations, *e.g*., cleansing, depilatories, face and neck, body and hand, foot powders and sprays, moisturizing, night preparations, paste masks, skin fresheners; and suntan preparations, *e.g*., gels, creams, and liquids, and indoor tanning preparations.

In some instances, the formulations, compositions, or preparations described herein, may comprise, be provided as, or disposed in at least one of a baby product, *e.g*., a baby shampoo, a baby lotion, a baby oil, a baby powder, a baby cream; a bath preparation, *e.g*., a bath oil, a tablet, a salt, a bubble bath, a bath capsule; a powder (dusting and talcum), a sachet; hair preparations, *e.g*., hair conditioners, rinses, shampoos, tonics, face powders, cuticle softeners, nail creams and lotions, oral hygiene products, mouthwashes, bath soaps, douches, feminine hygiene deodorants; shaving preparations, *e.g*., aftershave lotions, skin care preparations, *e.g*., cleansing, face and neck, body and hand, foot powders and sprays, moisturizing, night preparations, paste masks, skin fresheners; and suntan preparations, *e.g*., gels, creams, and liquids.

Other components may be added to pharmaceutical formulations, *e.g*., preparations, or cosmetic preparations as selected by one skilled in the art of cosmetic formulation such as, for example, water, mineral oil, coloring agent, perfume, aloe, glycerin, sodium chloride, sodium bicarbonate, pH buffers, UV blocking agents, silicone oil, natural oils, vitamin E, herbal concentrates, lactic acid, citric acid, talc, clay, calcium carbonate, magnesium carbonate, zinc oxide, starch, urea, and erythorbic acid, or any other excipient known by one of skill in the art, including those disclosed herein.

In some instances, the preparation may be disposed in, or provided as, a powder, cosmetic, cream, stick, aerosol, salve, wipe, or bandage.

In some instances, ammonia oxidizing bacteria is associated with a cream. The cream may be a fluid comprising a thickening agent, and generally has a consistency that allows it to be spread evenly on the skin. Exemplary creams include moisturizing lotion, face cream, and body lotion.

In some instances, the ammonia oxidizing bacteria is associated with a stick. A stick is typically a solid that, when placed in contact with a surface, transfers some of the stick contents to the surface. Exemplary sticks include deodorant stick, lipstick, lip balm in stick form, and sunscreen applicator sticks.

In some instances, the ammonia oxidizing bacteria is associated with an aerosol. An aerosol is typically a colloid of fine solid particles or fine liquid droplets, in a gas such as air. Aerosols may be created by placing the ammonia oxidizing bacteria (and optionally carriers) in a vessel under pressure, and then opening a valve to release the contents. The container may be designed to only exert levels of pressure that are compatible with ammonia oxidizing bacteria viability. For instance, the high pressure may be exerted for only a short time, and/or the pressure may be low enough not to impair viability. Examples of consumer uses of aerosols include for sunscreen, deodorant, perfume, hairspray, and insect repellant.

In some instances, the ammonia oxidizing bacteria is associated with a salve. A salve may be a topically applied agent with a liquid or cream-like consistency, intended to protect the skin or promote healing. Examples of salves include burn ointments and skin moisturizers.

In some instances, the ammonia oxidizing bacteria is associated with a wipe. A wipe may be a flexible material suitable for topically applying a liquid or cream onto skin. The wipe may be, *e.g*., paper-based or cloth based. Exemplary wipes include tissues and wet wipes.

The compositions comprising ammonia oxidizing bacteria may also comprise one or more of a moisturizing agent, deodorizing agent, scent, colorant, insect repellant, cleansing agent, or UV-blocking agent.

For instance, the moisturizing agent may be an agent that reduces or prevents skin dryness. Exemplary moisturizing agents include humectants (*e.g*., urea, glycerin, alpha hydroxy acids and dimethicone) and emollients (*e.g*., lanolin, mineral oil and petrolatum). Moisturizing agents may be included, *e.g*., in ammonia oxidizing bacteria-containing creams, balms, lotions, or sunscreen.

A deodorizing agent may be an agent that reduces unwanted odors. A deodorizing agent may work by directly neutralizing odors, preventing perspiration, or preventing the growth of odor-producing bacteria. Exemplary deodorizing agents include aluminum salts (*e.g*., aluminum chloride or aluminum chlorohydrate), cyclomethicone, talc, baking soda, essential oils, mineral salts, hops, and witch hazel. Deodorizing agents are typically present in spray or stick deodorants, and can also be found in some soaps and clothing.

An insect repellant may be an agent that can be applied to surfaces (e.g., skin) that discourage insects and other arthropods from lighting on the surface. Insect repellants include DEET (N,N-diethyl-m-toluamide), p-menthane-3,8-diol (PMD), icaridin, nepetalactone, citronella oil, neem oil, bog myrtle, dimethyl carbate, Tricyclodecenyl allyl ether, and IR3535 (3-[N-Butyl-N-acetyl]-aminopropionic acid, ethyl ester).

A cleansing agent may be an agent that removes dirt or unwanted bacteria from a surface like skin. Exemplary cleansing agents include bar soaps, liquid soaps, and shampoos.

A UV-blocking agent may be an agent that can be applied to a surface to reduce the amount of ultraviolet light the surface receives. A UV-blocking agent may block UV-A and/or UV-B rays. A UV blocking agent can function by absorbing, reflecting, or scattering UV. Exemplary UV-blocking agents include absorbers, *e.g*., homosalate, octisalate (also called octyl salicylate), octinoxate (also called octyl methoxycinnamate or OMC), octocrylene, oxybenzone, and avobenzone, and reflectors (*e.g*., titanium dioxide and zinc oxide). UV-blocking agents are typically presenst in sunscreens, and can also be found in skin creams and some cosmetics.

In some instances, ammonia oxidizing bacteria is associated with a conditioner. Conditioner generally refers to a substance with cream-like consistency that can be applied to hair to improve its appearance, strength, or manageability.

In some instances, ammonia oxidizing bacteria is associated with cloth. Cloth generally refers to a flexible material suitable to be made into clothing, *e.g*., having enough material strength to withstand everyday motion by a wearer. Cloth can be fibrous, woven, or knit; it can be made of a naturally occurring material or a synthetic material. Exemplary cloth materials include cotton, flax, wool, ramie, silk, denim, leather, nylon, polyester, and spandex, and blends thereof.

In some instances, ammonia oxidizing bacteria is associated with yarn. Yarn generally refers to a long, thin spun flexible material that is suitable for knitting or weaving. Yarn can be made of, *e.g*., wool, cotton, polyester, and blends thereof.

In some instances, ammonia oxidizing bacteria is associated with thread. Thread generally refers to a long, thin spun flexible material that is suitable for sewing. Thread generally has a thinner diameter than yarn. Thread can be made of, *e.g*., cotton, polyester, nylon, silk, and blends thereof.

Articles of clothing such as, for example, shoes, shoe inserts, pajamas, sneakers, belts, hats, shirts, underwear, athletic garments, helmets, towels, gloves, socks, bandages, and the like, may also be treated with ammonia oxidizing bacteria. Bedding, including sheets, pillows, pillow cases, and blankets may also be treated with ammonia oxidizing bacteria. In some instances, areas of skin that cannot be washed for a period of time may also be contacted with ammonia oxidizing bacteria. For example, skin enclosed in orthopedic casts which immobilize injured limbs during the healing process, and areas in proximity to injuries that must be kept dry for proper healing such as stitched wounds may benefit from contact with the ammonia oxidizing bacteria.

In some aspects, the present disclosure provides a wearable article comprising an ammonia oxidizing bacterium or ammonia oxidizing bacteria as described herein. A wearable article may be a light article that can be closely associated with a user's body, in a way that does not impede ambulation. Examples of wearable articles include a wristwatch, wristband, headband, hair elastic, hair nets, shower caps, hats, hairpieces, and jewelry. The wearable article comprising ammonia oxidizing bacteria described herein may provide, *e.g.,* at a concentration that provides one or more of a treatment or prevention of a skin disorder, a treatment or prevention of a disease or condition associated with low nitrite levels, a treatment or prevention of body odor, a treatment to supply nitric oxide to a subject, or a treatment to inhibit microbial growth.

In some instances, the ammonia oxidizing bacteria is associated with a product intended to contact the hair, for example, a brush, comb, shampoo, conditioner, headband, hair elastic, hair nets, shower caps, hats, and hairpieces. Nitric oxide formed on the hair, away from the skin surface, may be captured in a hat, scarf or face mask and directed into inhaled air.

Articles contacting the surface of a human subject, such as a diaper, may be associated with ammonia oxidizing bacteria. Because diapers are designed to hold and contain urine and feces produced by incontinent individuals, the urea in urine and feces can be hydrolyzed by skin and fecal bacteria to form free ammonia which is irritating and may cause diaper rash. Incorporation of bacteria that metabolize urea into nitrite or nitrate, such as ammonia oxidizing bacteria, may avoid the release of free ammonia and may release nitrite and ultimately NO which may aid in the maintenance of healthy skin for both children and incontinent adults. The release of nitric oxide in diapers may also have anti-microbial effects on disease causing organisms present in human feces. This effect may continue even after disposable diapers are disposed of as waste and may reduce the incidence of transmission of disease through contact with soiled disposable diapers.

In some instances, the product comprising ammonia oxidizing bacteria is packaged. The packaging may serve to compact the product or protect it from damage, dirt, or degradation. The packaging may comprise, *e.g*., plastic, paper, cardboard, or wood. In some instances the packaging is impermeable to bacteria. In some instances the packaging is permeable to oxygen and/or carbon dioxide.

### 9. Methods of treatment with ammonia oxidizing bacteria

The present disclosure provides various methods of treating diseases and conditions using ammonia oxidizing bacteria, *e.g*., by administering ammonia oxidizing bacteria, e.g., a preparation of ammonia oxidizing bacteria, *e.g*., a natural product or a fortified natural product, or compositions, preparations, or formulations comprising a natural product or a fortified natural product. The subject may be evaluated, *e.g*., evaluated for application of ammonia oxidizing bacteria, *e.g*., prior to administering the preparation of ammonia oxidizing bacteria to the subject.

The ammonia oxidizing bacteria that may be used to treat diseases and conditions include all the ammonia oxidizing bacteria compositions described in this application, *e.g*. a preparation of ammonia oxidizing bacteria, a natural product or a fortified natural product, or compositions, preparations, or formulations comprising a natural product or a fortified natural product.

For instance, the disclosure provides uses, for treating a condition or disease (*e.g*., inhibiting microbial growth on a subject's skin), a composition of ammonia oxidizing bacteria. The ammonia oxidizing bacteria may be used to treat an ulcer, e.g., venous ulcer, e.g., leg ulcer, e.g., venous leg ulcer, *e.g*., diabetic ulcers, *e.g*., diabetic foot ulcers, chronic wounds, acne, e.g., acne vulgaris, rosacea, eczema, uticaria, or psoriasis.

The systems and methods of the present disclosure may provide for, or contain contents, to be useful for treating or preventing a skin disorder, treating or preventing a disease or condition associated with low nitrite levels, a treating or preventing body odor, treating to supply nitric oxide to a subject, or treating to inhibit microbial growth.

The systems and methods of the present disclosure may provide for reducing an amount of undesirable bacteria from an environment, *e.g*., a surface of a subject.

The systems and methods of the present disclosure may provide for, or contain contents, to be useful in a treatment of at least one of HIV dermatitis, infection in an ulcer, e.g., venous ulcer, e.g., leg ulcer, e.g., venous leg ulcer, e.g. infection in a diabetic foot ulcer, atopic dermatitis, acne, e.g., acne vulgaris, eczema, contact dermatitis, allergic reaction, psoriasis, uticaria, rosacea, skin infections, vascular disease, vaginal yeast infection, a sexually transmitted disease, heart disease, atherosclerosis, baldness, leg ulcers secondary to diabetes or confinement to bed, angina, particularly chronic, stable angina pectoris, ischemic diseases, congestive heart failure, myocardial infarction, ischemia reperfusion injury, laminitis, hypertension, hypertrophic organ degeneration, Raynaud's phenomenon, fibrosis, fibrotic organ degeneration, allergies, autoimmune sensitization, end stage renal disease, obesity, impotence, pneumonia, primary immunodeficiency, epidermal lysis bulosa, or cancer.

The systems and methods of the present disclosure may provide for, or contain contents, to be useful in a treatment of at least one of acne, e.g., acne vulgaris, eczema, psoriasis, uticaria, rosacea, skin infections and wounds, *e.g*., an infected wound.

In some instances, ammonia oxidizing bacteria are used to treat a subject. Subjects may include an animal, a mammal, a human, a non-human animal, a livestock animal, or a companion animal.

In some instances, ammonia oxidizing bacteria described herein are used to inhibit the growth of other organisms. For instance, ammonia oxidizing bacteria may be well-adapted for long-term colonization of human skin, and in some instances it out-competes other bacteria that are undesirable on the skin. Undesirable skin bacteria include, *e.g.*, those that can infect wounds, raise the risk or severity of a disease, or produce odors. Undesirable bacteria may be referred to as pathogenic bacteria. Certain undesirable skin bacteria include *Staphylococcus aureus* (*S. aureus*), *Psuedonomas aeruginosa* (*P. aeruginosa*), *Streptococcus pyogenes* (*S. pyogenes*)*, Acinetobacter baumannii* (*A. baumannii*), *Propionibacteria*, and *Stenotrophomonas.* The ammonia oxidizing bacteria described herein may out-compete other organisms by, *e.g*., consuming scarce nutrients, or generating byproducts that are harmful to other organisms, *e.g*., changing the pH of the skin to a level that is not conducive to the undesirable organism's growth.

Accordingly, the present disclosure provides, *inter alia,* a method of inhibiting microbial growth on a subject's skin, comprising topically administering to a human in need thereof an effective dose of ammonia oxidizing bacteria as described herein. Similarly, the present disclosure provides ammonia oxidizing bacteria as described herein for use in inhibiting microbial growth on a subject's skin. Likewise, the present disclosure provides a use of ammonia oxidizing bacteria in the manufacture of a medicament for inhibiting microbial growth on a subject's skin.

The present disclosure also provides a method of supplying nitric oxide to a subject, comprising positioning an effective dose of ammonia oxidizing bacteria described herein in close proximity to the subject. Similarly, the present disclosure provides ammonia oxidizing bacteria as described herein for use in supplying nitric oxide to a subject. Likewise, the present disclosure provides a use of ammonia oxidizing bacteria in the manufacture of a medicament or composition suitable for position in close proximity to a subject.

The present disclosure also provides a method of reducing body odor, comprising topically administering to a subject in need thereof an effective dose of ammonia oxidizing bacteria described herein. Similarly, the present disclosure provides ammonia oxidizing bacteria as described herein for use in reducing body odor in a subject. Likewise, the present disclosure provides a use of ammonia oxidizing bacteria as described herein in the manufacture of a medicament or composition for reducing body odor.

The present disclosure also provides a method of treating or preventing a disease associated with low nitrite levels, comprising topically administering to a subject in need thereof a therapeutically effective dose of ammonia oxidizing bacteria described herein. Similarly, the present disclosure provides a topical formulation of ammonia oxidizing bacteria as described herein for use in treating a disease associated with low nitrite levels. Likewise, the present disclosure provides a use of ammonia oxidizing bacteria as described herein in the manufacture of a topical medicament for treating a disease associated with low nitrite levels.

The present disclosure also provides a method of treating or preventing a skin disorder or skin infection, comprising topically administering to a subject in need thereof a therapeutically effective dose of ammonia oxidizing bacteria as described herein. Similarly, the present disclosure provides ammonia oxidizing bacteria as described herein for use in treating a skin disorder in a subject. Likewise, the present disclosure provides a use of ammonia oxidizing bacteria as described herein in the manufacture of a medicament for treating skin disorder. In instances, the skin disorder is acne, e.g., acne vulgaris, rosacea, eczema, psoriasis, or urticaria; the skin infection is impetigo.

While not wishing to be bound by theory, it is proposed that treatment of acne e.g., acne vulgaris, with a therapeutically effective dose of ammonia oxidizing bacteria; and/or limiting and/or inhibiting the spread and proliferation of Propionibacterium acnes associated with acne vulgaris through acidified nitrite and NO production.

While not wishing to be bound by theory, it is proposed that treatment of rosacea with a therapeutically effective dose of ammonia oxidizing bacteria as described herein may involve downregulation due to NO generation. This may be due to expression of Kazal-type KLK5/KLK7 inhibitor(s) that may reduce formation of the human cathelicidin peptide LL-37 from its precursor propeptide hCAP18.

While not wishing to be bound by theory, it is proposed that treatment of eczema and/or atopic dermatitis with a therapeutically effective dose of ammonia oxidizing bacteria as described herein may involve donwregulation of inflammation due to NO generation; and/or limiting and/or inhibiting the spread and proliferation of *S. aureus* and other skin pathogens often associated with very high colonization rates and skin loads in atopic dermatitis through acidified nitrite and NO production.

While not wishing to be bound by theory, it is proposed that treatment of psoriasis with a therapeutically effective dose of ammonia oxidizing bacteria described herein may involve downregulation of inflammation due to NO generation and reduction in formation of human cathelicidin peptide LL-37.

While not wishing to be bound by theory, it is proposed that treatment of psoriasis with a therapeutically effective dose of ammonia oxidizing bacteria as described herein may involve downregulation of inflammation due to NO generation.

While not wishing to be bound by theory, it is proposed that treatment of impetigo or other skin and soft tissue infections with a therapeutically effective dose of ammonia oxidizing bacteria bacteria as described herein may involve limiting and/or inhibiting the spread and proliferation of *Staphylococcus aureus* (*S. aureus*)*, Psuedonomas aeruginosa* (*P. aeruginosa*)*, Streptococcus pyogenes* (*S. pyogenes*), *Acinetobacter baumannii* (*A. baumannii*), *Propionibacteria*, and *Stenotrophomonas.*

The present disclosure also provides a method of promoting wound healing, comprising administering to a wound an effective dose of ammonia oxidizing bacteria as described herein. Similarly, the present disclosure provides ammonia oxidizing bacteria as described herein for use in treating a wound. Likewise, the present disclosure provides a use of ammonia oxidizing bacteria as described herein in the manufacture of a medicament or a composition for treating a wound.

Ammonia oxidizing bacteria as described herein may be used to promote wound healing in a patient that has an impaired healing ability, *e.g*., a diabetic patient.

In some instances, this disclosure provides methods of using ammonia oxidizing bacteria as described herein to prevent a disease or disorder, *e.g*., a skin disorder. Prevention, in certain instances, means reducing the risk of a subject developing a disease, compared to a similar untreated subject. The risk need not be reduced to zero.

Individuals having a reduced bathing frequency, such as astronauts, submarine crew members, military personnel during a campaign, civilian workers in remote locations, refugees, bedridden individuals and many others may maintain healthier skin by maintaining ammonia oxidizing bacteria on the skin. With regard to bedridden individuals, the ammonia oxidizing bacteria in some instances reduces the frequency or severity of bed sores by augmenting inadequate circulation.

It is appreciated that many modern degenerative diseases may be caused by a lack of NO species, and that ammonia oxidizing bacteria on the external skin can supply those species by diffusion, and that application of ammonia oxidizing bacteria to the skin resolves long standing medical conditions. In certain instances, ammonia oxidizing bacteria are applied to a subject to offset modern bathing practices, especially with anionic detergents remove ammonia oxidizing bacteria from the external skin.

One suitable method of topical application to apply sufficient ammonia oxidizing bacteria and then wear sufficient clothing so as to induce sweating. However, many people will want to derive the benefits of ammonia oxidizing bacteria while maintaining their current bathing habits, in which case, a culture of the bacteria can be applied along with sufficient substrate for them to produce NO. A nutrient solution approximating the inorganic composition of human sweat can be used for this purpose. Using bacteria adapted to media approximating human sweat minimizes the time for them to adapt when applied. Since sweat evaporates once excreted onto the skin surface, using a culture media that has a higher ionic strength is desirable. A concentration approximately twice that of human sweat is suitable, but other conditions are also contemplated. Ammonia oxidizing bacteria's nutritional needs are typically met with NH₃ or urea, O₂, CO₂, and minerals. In some instances, the substrate comprises trace minerals including iron, copper, zinc, cobalt, molybdenum, manganese, sodium, potassium, calcium, magnesium, chloride, phosphate, sulfate, or any combination thereof.

In some instances, the present disclosure provides a method of treating a wound by applying a bandage comprising ammonia oxidizing bacteria to the wound. Also provided are methods of producing such a bandage. The bandage may comprise, for example, an adhesive portion to affix the bandage to undamaged skin near the wound and a soft, flexible portion to cover or overlay the wound. In some instances, the bandage contains no other organisms but ammonia oxidizing bacteria. The bandage may made of a permeable material that allows gasses like oxygen and carbon dioxide to reach the ammonia oxidizing bacteria when the bandage is applied to the wound. In certain instances, the bandage comprises nutrients for ammonia oxidizing bacteria such as ammonium, ammonia, urea, or trace minerals. In certain instances, the bandage comprises an antibiotic to which the ammonia oxidizing bacteria is resistant. The antibiotic resistance may arise from one or more endogenous resistance gene or from one or more transgenes.

In some instances, the ammonia oxidizing bacteria *e.g*., a preparation of ammonia oxidizing bacteria, is administered at a dose of about 10⁸ - 10⁹ CFU, 10⁹ - 10¹⁰ CFU, 10¹⁰ - 10¹¹ CFU, 10¹¹-10¹² CFU, 10¹²-10¹³ CFU, or 10¹³-10¹⁴ CFU per application or per day. In some instances, the ammonia oxidizing bacteria is administered topically at a dose of about 10⁹-10¹⁰ CFU, about 1 x 10⁹ - 5 x 10⁹, 1 x 10⁹ - 3 x 10⁹, or 1 x 10⁹ - 10 x 10⁹ CFU; or about 10¹⁰-10¹¹ CFU, *e.g*., about 1 x 10¹⁰ - 5 x 10¹⁰, 1 x 10¹⁰ - 3 x 10¹⁰, or 1 x 10¹⁰ - 2 x 10¹⁰ CFU; or about 10¹¹-10¹² CFU, *e.g*., about 1 x 10¹¹ - 5 x 10¹¹, 1 x 10¹¹ - 3 x 10¹¹, or 1 x 10¹¹ - 2 x 10¹¹ CFU; or about 10¹²-10¹³ CFU, *e.g*., about 1 x 10¹² - 5 x 10¹², 1 x 10¹² - 3 x 10¹², or 1 x 10¹² - 2 x 10¹² CFU; or about 10¹³-10¹⁴ CFU, *e.g*., about 1 x 10¹³ - 5 x 10¹³, 1 x 10¹³ - 3 x 10¹³, or 1 x 10¹³ - 2 x 10¹³ CFU.

In some instances, the ammonia oxidizing bacteria is administered in a volume of about 1-2, 2-5, 5-10, 10-15, 12-18, 15-20, 20-25, or 25-50 ml per dose. In some instances, the solution is at a concentration of about 10⁸-10⁹, 10⁹-10¹⁰, or 10¹⁰-10¹¹ CFUs/ml. In some instances, the ammonia oxidizing bacteria is administered as two 15 ml doses per day, where each dose is at a concentration of 10⁹ CFU/ml.

In some instances, the ammonia oxidizing bacteria is administered once, twice, three, or four times per day. In some instances, the ammonia oxidizing bacteria is administered once, twice, three, four, five, or six times per week. In some instances, the ammonia oxidizing bacteria is administered shortly after bathing. In some instances, the ammonia oxidizing bacteria is administered shortly before sleep.

In some instances, the ammonia oxidizing bacteria is administered for about 1-3, 3-5, 5-7, 7-9, 5-10, 10-14, 12-18, 12-21, 21-28, 28-35, 35-42, 42-49, 49-56, 46-63, 63-70, 70-77, 77-84, 84-91 days, *e.g*., for about 1 month, for about 2 months, for about 3 months. In some instances, the ammonia oxidizing bacteria is administered for an indefinite period of time, e.g., greater than one year, greater than 5 years, greater than 10 years, greater than 15 years, greater than 30 years, greater than 50 years, greater than 75 years.
In certain aspects, the present disclosure provides combination therapies comprising ammonia oxidizing bacteria and a second therapeutic. For instance, the disclosure provides physical admixtures of the two (or more) therapies are physically admixed. In other instances, the two (or more) therapies are administered in combination as separate formulation. The second therapy may be, *e.g*., a pharmaceutical agent, surgery, or any other medical approach that treats the relevant disease or disorder. The following paragraphs describe combination therapies capable of treating ulcers, e.g., diabetic ulcers, chronic wounds, acne, e.g., acne vulgaris, rosacea, eczema, and psoriasis. The combination therapy may treat a venous leg ulcer.

For instance, in a combination therapy capable of treating ulcers, e.g., venous ulcer, e.g., leg ulcer, e.g., venous leg ulcer, e.g., the second therapy may comprise, *e.g*., a wound dressing (*e.g*., absorptive fillers, hydrogel dressings, or hydrocolloids), angiotensin, angiotensin analogues, platelet-rich fibrin therapy, hyperbaric oxygen therapy, negative pressure wound therapy, debridement, drainage, arterial revascularization, hyperbaric oxygen therapy, low level laser therapy, and gastrocnemius recession. The combination therapy may comprise one or more of the above-mentioned treatments.

In a combination therapy capable of treating chronic wounds, the second therapy may comprise, *e.g*., an antibiotic (*e.g*., topical or systemic, and bacteriocidal or bacteriostatic) such as Penicillins, cephalosporins, polymyxins, rifamycins, lipiarmycins, quinolones, sulfonamides, macrolides, lincosamides, tetracyclines, cyclic lipopeptides, glycylcyclines, oxazolidinones, and lipiarmycins; angiotensin, angiotensin analogues; debridement; drainage; wound irrigation; negative pressure wound therapy; application of heat; arterial revascularization; hyperbaric oxygen therapy; antioxidants such as ascorbic acid, glutathione, lipoic acid, carotenes, α-tocopherol, or ubiquinol; low level laser therapy; gastrocnemius recession; growth factors such as vascular endothelial growth factor, insulin-like growth factor 1-2, platelet derived growth factor, transforming growth factor-β, or epidermal growth factor; application of autologous platelets such as those that secrete one or more growth factors such as vascular endothelial growth factor, insulin-like growth factor 1-2, platelet derived growth factor, transforming growth factor-β, or epidermal growth factor; implantation of cultured keratinocytes; allograft; collagen, for instance a dressing comprising collagen; or protease inhibitors such as SLPI. The combination therapy may comprise one or more of the above-mentioned treatments.

In a combination therapy capable of treating acne, e.g., acne vulgaris, the second therapy may comprise, *e.g*., a medication (*e.g*., systemic or topical) such as Benzoyl peroxide, antibiotics (such as erythromycin, clindamycin, or a tetracycline), Salicylic acid, hormones (*e.g*., comprising a progestin such as desogestrel, norgestimate or drospirenone), retinoids such as tretinoin, adapalene, tazarotene, or isotretinoin. The second therapy may also be a procedure such as comedo extraction, corticosteroid injection, or surgical lancing. The combination therapy may comprise one or more of the above-mentioned treatments.

In a combination therapy capable of treating rosacea, the second therapy may comprise, *e.g*., an antibiotic, *e.g*., an oral tetracycline antibiotic such as tetracycline, doxycycline, or minocycline, or a topical antibiotic such as metronidazole; azelaic acid; alpha-hydroxy acid; isotretinoin can be prescribed; sandalwood oil; clonidine; beta-blockers such as nadolol and propranolol; antihistamines (such as loratadine); mirtazapine; methylsulfonylmethane or silymarin, optionally in combination with each other; lasers such as dermatological vascular laser or CO₂ laser; or light therapies such as intense pulsed light, low-level light therapy or photorejuvenation. The combination therapy may comprise one or more of the above-mentioned treatments.

In a combination therapy capable of treating eczema, the second therapy may comprise, *e.g*., a corticosteroid such as hydrocortisone or clobetasol propionate, immunosuppressants (topical or systemic) such as pimecrolimus, tacrolimus, ciclosporin, azathioprine or methotrexate, or light therapy such as with ultraviolet light. The combination therapy may comprise one or more of the above-mentioned treatments.

In a combination therapy capable of treating psoriasis, the second therapy may comprise, *e.g.,* a corticosteroid such as desoximetasone; a retinoid; coal tar; Vitamin D or an analogue thereof such as paricalcitol or calcipotriol; moisturizers and emollients such as mineral oil, vaseline, calcipotriol, decubal , or coconut oil; dithranol; or fluocinonide. The combination therapy may comprise one or more of the above-mentioned treatments.

### 10. Experimental models for refining ammonia oxidizing bacteria treatments

Treatments comprising ammonia oxidizing bacteria as described herein (optionally in combination with another therapy) can be refined using a number of model systems. These model systems can be used to determine suitable doses and timing of administration.

For instance, with respect to chronic wounds and ulcers, e.g., venous ulcers, e.g., diabetic ulcers, or other ulcers disclosed herein,, one may use the mouse skin puncture model. Other models for these disorders include controlled cutaneous ischemia in a guinea pig model, rabbit ear ulcer model, application of calcium to a wound, or topical application of doxorubicin.

With respect to acne, e.g., acne vulgaris, one may use (for example) the Mexican hairless dog model, the Rhino mouse model, or the rabbit ear assay. With respect to rosacea, one may use (for example) intradermal injection of LL-37 into mouse skin or the Syrian hamster model. With respect to eczema, one may use (for example) application of a crude extract of Dermatophagoides farina, application of dinitrochlorobenzene to the ears of sensitized guinea pigs, or NC/Nga mice. With respect to psoriasis, one may use (for example) xenograft models in which involved and uninvolved psoriatic skin are transplanted onto immunodeficient mice, application of an antibody directed against interleukin 15 to the skin of SCID mice, and the Sharpin^{cpdm}/Sharpin^{cpdm} mouse model.

### 11. Mechanism of therapeutic benefit

While not wishing to be bound by theory, it is believed that one or more of the following mechanisms contributes to the beneficial effect of ammonia oxidizing bacteria are found in International Application WO/2005/030147.

In order to understand the beneficial aspects of these bacteria, it is helpful to understand angiogenesis. All body cells, except those within a few hundred microns of the external air, receive all metabolic oxygen from the blood supply. The oxygen is absorbed by the blood in the lung, is carried by red blood cells as oxygenated hemoglobin to the peripheral tissues, where it is exchanged for carbon dioxide, which is carried back and exhaled from the lung. Oxygen must diffuse from the erythrocyte, through the plasma, through the endothelium and through the various tissues until it reached the mitochondria in the cell which consumes it. The human body contains about 5 liters of blood, so the volume of the circulatory system is small compared to that of the body. Oxygen is not actively transported. It passively diffuses down a concentration gradient from the air to the erythrocyte, from the erythrocyte to the cell, and from the cell to cytochrome oxidase where it is consumed. The concentration of oxygen at the site of consumption is the lowest in the body, and the O₂ flux is determined by the diffusion resistance and the concentration gradient. Achieving sufficient oxygen supply to all the peripheral tissues requires exquisite control of capillary size and location. If the spacing between capillaries were increased, achieving the same flux of oxygen would require a larger concentration difference and hence a lower O₂ concentration at cytochrome oxidase. With more cells between capillaries, the O₂ demand would be greater. If the spacing between capillaries were decreased, there would be less space available for the cells that perform the metabolic function of the organ.

In certain aspects, it is appreciated that NO from ammonia oxidizing bacteria is readily absorbed by the outer skin and converted into S-nitrosothiols since the outer skin is free from hemoglobin. M. Stucker et al. have shown that the external skin receives all of its oxygen from the external air in "The cutaneous uptake of atmospheric oxygen contributes significantly to the oxygen supply of human dermis and epidermis. (Journal of Physiology (2002), 538.3, pp. 985-994.) This is readily apparent, because the external skin can be seen to be essentially erythrocyte free. There is circulation of plasma through these layers because they are living and do require the other nutrients in blood, just not the oxygen. S-nitrosothiols formed are stable, can diffuse throughout the body, and constitute a volume source of authentic NO and a source of NO to transnitrosate protein thiols.

In some aspects, it is appreciated that capillary rarefaction may be one of the first indications of insufficient levels of NO. F. T. Tarek et al. have shown that sparse capillaries, or capillary rarefaction, is commonly seen in people with essential hypertension. (Structural Skin Capillary Rarefaction in Essential Hypertension. Hypertension. 1999;33:998-1001).

A great many conditions are associated with the capillary density becoming sparser. Hypertension is one, and researchers reported that sparse capillaries are also seen in the children of people with essential hypertension, and also in people with diabetes. Significant complications of diabetes are hypertension, diabetic nephropathy, diabetic retinopathy, and diabetic neuropathy. R. Candido et al. have found that the last two conditions are characterized by a reduction in blood flow to the affected areas prior to observed symptoms. (Haemodynamics in microvascular complications in type 1 diabetes. Diabetes Metab Res Rev 2002; 18: 286-304.) Reduced capillary density is associated with obesity, and simple weight loss increases capillary density as shown by A Philip et al. in "Effect of Weight Loss on Muscle Fiber Type, Fiber Size, Capilarity, and Succinate Dehydrogenase Activity in Humans. The Journal of Clinical Endocrinology & Metabolism Vol. 84, No. 11 4185-4190, 1999.

Researchers have shown that in primary Raynaud's phenomena (PRP), the nailfold capillaries are sparser (slightly) than in normal controls, and more abundant than in patients that have progressed to systemic sclerosis (SSc). M. Bukhari, Increased Nailfold Capillary Dimensions In Primary Raynaud's Phenomenon And Systemic Sclerosis. British Journal of Rheumatology, Vol. 24 No 35: 1127-1131, 1996. They found that the capillary density decreased from 35 loops/mm² (normal controls) to 33 (PRP), to 17 (SSc). The average distance between capillary limbs was 18µ, 18µ, and 30µ for controls, PRP and SSc, respectively.

In certain aspects, it is appreciated that the mechanism that the body normally uses to sense "hypoxia" may affect the body's system that regulates capillary density. According to this aspect of the disclosure, a significant component of "hypoxia" is sensed, not by a decrease in O2 levels, but rather by an increase in NO levels. Lowering of basal NO levels interferes with this "hypoxia" sensing, and so affects many bodily functions regulated through "hypoxia." For Example, anemia is commonly defined as "not enough hemoglobin," and one consequence of not enough hemoglobin is "hypoxia", which is defined as "not enough oxygen." According to some aspects, these common definitions do not account for the nitric oxide mediated aspects of both conditions.

At rest, acute isovolemic anemia is well tolerated. A 2/3 reduction in hematocrit has minimal effect on venous return PvO2, indicating no reduction in either O₂ tension or delivery throughout the entire body. Weiskopf et al. Human cardiovascular and metabolic response to acute, severe isovolemic anemia. JAMA 1998, vol 279, No. 3, 217-221. At 50% reduction (from 140 to 70g Hb/L), the average PvO2 (over 32 subjects) declined from about 77% to about 74% (of saturation). The reduction in O₂ capacity of the blood is compensated for by vasodilatation and tachycardia with the heart rate increasing from 63 to 85 bpm. That the compensation is effective is readily apparent, however, the mechanism is not. A typical explanation is that "hypoxia" sensors detected "hypoxia" and compensated with vasodilatation and tachycardia. However, there was no "hypoxia" to detect. There was a slight decrease in blood lactate (a marker for anaerobic respiration) from 0.77 to 0.62 mM/L indicating less anaerobic respiration and less "hypoxia." The 3% reduction in venous return PvO2 is the same level of "hypoxia" one would get by ascending 300 meters in altitude (which typically does not produce tachycardia). With the O₂ concentration in the venous return staying the same, and the O₂ consumption staying the same, there is no place in the body where there is a reduction in O₂ concentration. Compensation during isovolemic anemia may not occur because of O₂ sensing.

Thus the vasodilatation that is observed in acute isovolemic anemia may be due to the increased NO concentration at the vessel wall. NO mediates dilatation of vessels in response to shear stress and other factors. No change in levels of NO metabolites would be observed, because the production rate of NO is unchanged and continues to equal the destruction rate. The observation of no "hypoxic" compensation with metHb substitution can be understood because metHb binds NO just as Hb does, so there is no NO concentration increase with metHb substitution as there is with Hb withdrawal.

Nitric oxide plays a role in many metabolic pathways. It has been suggested that a basal level of NO exerts a tonal inhibitory response, and that reduction of this basal level leads to a dis-inhibition of those pathways. Zanzinger et al. have reported that NO has been shown to inhibit basal sympathetic tone and attenuate excitatory reflexes. (Inhibition of basal and reflex-mediated sympathetic activity in the RVLM by nitric oxide. Am. J. Physiol. 268 (Regulatory Integrative Comp. Physiol. 37): R958-R962, 1995.)

In some aspects, it is appreciated that one component of a volume source of NO is low molecular weight S-nitrosothiols produced in the erythrocyte free skin from NO produced on the external skin by ammonia oxidizing bacteria. These low molecular weight S-nitrosothiols are stable for long periods, and can diffuse and circulate freely in the plasma. Various enzymes can cleave the NO from various S-nitrosothiols liberating NO at the enzyme site. It is the loss of this volume source of NO from AOB on the skin that leads to disruptions in normal physiology. The advantage to the body of using S-nitrosothiols to generate NO far from a capillary is that O₂ is not required for NO production from S-nitrosothiols. Production of NO from nitric oxide synthase (NOS) does require O₂. With a sufficient background of S-nitrosothiols, NO can be generated even in anoxic regions. Free NO is not needed either since NO only exerts effects when attached to another molecule, such as the thiol of a cysteine residue or the iron in a heme, so the effects of NO can be mediated by transnitrosation reactions even in the absence of free NO provided that S-nitrosothiols and transnitrosation enzymes are present.

Frank et al. have shown that the angiogenesis that accompanies normal wound healing is produced in part by elevated VEGF which is induced by increased nitric oxide. (Nitric oxide triggers enhanced induction of vascular endothelial growth factor expression in cultured keratinocytes (HaCaT) and during cutaneous wound repair. FASEB J. 13, 2002-2014 (1999).)

NO has a role in the development of cancer, indicating that the bacteria described herein may be used in methods of cancer treatment and prevention. According to certain aspects, it is appreciated that the presence of NO during hypoxia may prevent cells from dividing while under hypoxic stress, when cells are at greater risk for errors in copying DNA. One relevant cell function is the regulation of the cell cycle. This is the regulatory program which controls how and when the cell replicates DNA, assembles it into duplicate chromosomes, and divides. The regulation of the cell cycle is extremely complex, and is not fully understood. However, it is known that there are many points along the path of the cell cycle where the cycle can be arrested and division halted until conditions for doing so have improved. The p53 tumor suppressor protein is a key protein in the regulation of the cell cycle, and it serves to initiate both cell arrest and apoptosis from diverse cell stress signals including DNA damage and p53 is mutated in over half of human cancers as reported by Ashcroft et al. in "Stress Signals Utilize Multiple Pathways To Stabilize p53." (Molecular And Cellular Biology, May 2000, p. 3224-3233.) Hypoxia does initiate accumulation of p53, and while hypoxia is important in regulating the cell cycle, hypoxia alone fails to induce the downstream expression of p53 mRNA effector proteins and so fails to cause arrest of the cell cycle. Goda et al. have reported that hypoxic induction of cell arrest requires hypoxia-inducing factor-1 (HIF-1α). (Hypoxia-Inducible Factor 1α Is Essential for Cell Cycle Arrest during Hypoxia. Molecular And Cellular Biology, Jan. 2003, p. 359-369.) Britta et al. have reported that NO is one of the main stimuli for HIF-1α. (Accumulation of HIF-1a under the influence of nitric oxide. Blood, 15 February 2001, Volume 97, Number 4.) In contrast, NO does cause the accumulation of transcriptionally active p53 and does cause arrest of the cell cycle and does cause apoptosis. Wang et al., P53 Activation By Nitric Oxide Involves Down-Regulation Of Mdm2. THE JOURNAL OF BIOLOGICAL CHEMISTRY Vol. 277, No. 18, Issue Of May 3, Pp. 15697-15702, 2002.

In certain aspect of the disclosure, it is appreciated that preventing the necrotic death of cells by preventing the capillary rarefaction that leads to their hypoxic death may prevent autoimmune disorders. When cells are exposed to chronic hypoxia, the production of reactive oxygen species (ROS) is increased, and there is increased damage to the cells metabolic machinery and ultimately to the cells' DNA. Decreased metabolic capacity will decrease capacity for repair of damage due to ROS and due to exogenous carcinogen exposure. Over time, the damage accumulates and increases the chance of three events: the cell will undergo deletion of cancer-preventing genes and the cell will become cancerous, the cell will die through necrosis, or the cell will die through apoptosis. When cells die, either through necrosis or apoptosis, the cell debris must be cleared from the site. Dead cells are phagocytosed by immune cells, including dendritic cells and macrophages. When these cells phagocytose a body, it is digested by various proteolytic enzymes into antigenic fragments, and then these antigens are attached to the major histocompatability complex (MHC1, MHC2) and the antigen-MHC complex is moved to the surface of the cell where it can interact with T cells and activate the T cells in various ways. Any cell injury releases adjuvants which stimulate the immune system in various ways. In general, cells that undergo necrosis stimulate a greater immune response than cells that undergo apoptosis. Chronic exposure of immune cells to dead and dying cells is therefore likely to lead to autoimmune disorders.

In certain aspects, it is appreciated that low basal NO leads to fibrotic hypertrophy. Once a dead cell has been cleared, a new cell cannot easily take its place, because there is insufficient O₂ to support it. Any such new cell would suffer the same fate. The space can remain empty, in which case the organ shrinks, the capillaries draw closer together, new cells are now deprived of the VEGF formerly produced by the now-missing cell, so capillaries ablate and the hypoxic zone reforms. This could result in a general shrinkage of the affected tissues. In tissues that support fibrosis, relatively inert collagen fibers can fill the space. Since the metabolic requirements of the body for the particular organ in question are not reduced, the organ may attempt to grow larger, but now with a significant fibrous content. This may result in fibrotic hypertrophy, such as of the heart and liver. Some organs, such as the brain, cannot grow larger or smaller because the three-dimensional connectivity of nerves and blood vessels are important, and cannot be continuously and simultaneously mapped onto an asymmetrically shrinking brain. The space must be filled with something, and β-amyloid might be the (not so inert) space filler. The kidney cannot grow larger because of the renal capsule, so the number of living cells becomes smaller and they are replaced with fibrotic tissue. If the dead cells are cleared, the tissue shrinks, and the ratio of NO/O₂ goes down again, and the capillaries again become sparser. This may set up the vicious circle of end stage renal disease, congestive heart failure/cardiac hypertrophy, primary biliary cirrhosis, Alzheimer's disease, atherosclerosis, inflammatory bowel disease, hypertrophic scar formation, and the multiple connective tissue diseases starting with Raynaud's phenomena and ending with Systemic Sclerosis and primary Sjogren's syndrome where capillary rarefaction is also observed. Ferrini et al, have shown that a reduction in basal NO levels through chronic inhibition of NOS with L-NAME leads to generalized fibrosis of the heart and kidneys. (Antifibrotic Role of Inducible Nitric Oxide Synthase. Nitirc Oxide: Biology and Chemistry Vol. 6, No. 3, pp. 283-294 (2002).) It may be that low basal NO leads to fibrotic hypertrophy.

In certain aspects, it is appreciated that capillary rarefaction affects a subject's ability to control their appetite. Capillary rarefaction is observed in the brains of aged humans and animals. Capillary rarefaction is associated with declines in circulating growth factors including insulin like growth factor-1. Neurogenesis in the adult brain is coordinated with angiogenesis. Since the brain regulates many homeostatic functions, increased diffusion lengths between capillaries to control elements of the brain might be "interpreted" as inadequate blood concentrations of those species. The flux of glucose in the brain is quite close to normal metabolic needs, where glucose flux is only 50 to 75% greater than glucose consumption and the glucose transporters across the blood brain barrier are saturable, steriospecific and independent of energy or ion gradients. A large part of the regulation of appetite is mediated through the brain, and capillary rarefaction may cause an adequate blood concentration of "nutrients" (or marker compounds proportional to "nutrients") to be interpreted as insufficient. This may be one cause of obesity.

According to certain aspects, it is appreciated that capillary rarefaction may be a cause of non-insulin dependent diabetes. Non-insulin dependent diabetes (NIDDM) is also known as the Metabolic Syndrome or Diabetes type 2, and is characterized by insulin resistance. The sensitivity of the body to insulin is reduced, and insulin levels increase People with NIDDM have high blood glucose, high blood triglycerides, are typically obese, hypertensive, and typically have significant visceral fat.

Other symptoms accompany NIDDM, which may point to capillary rarefaction as the cause. In a study of 40 men, with and without NIDDM, obese (BMI 29) and lean (BMI 24) (10 of each), Konrad et al. report that blood lactate levels at rest were 1.78, 2.26, 2.42, and 2.76 (mM/L) for lean men without, obese men without, lean men with NIDDM, obese men with NIDDM respectively. (A-Lipoic acid treatment decreases serum lactate and pyruvate concentrations and improves glucose effectiveness in lean and obese patients with type 2 diabetes. Diabetes Care 22:280-287, 1999.) Lactate is a measure of anaerobic glycolysis. When O₂ is insufficient to generate ATP through oxidative phosphorylation, cells can produce ATP through anaerobic glycolysis. One of the products of anaerobic glycolysis is lactate, which must be exported from the cells, otherwise the pH drops and function is compromised. Blood lactate is commonly measured in exercise studies, where an increase indicates the work load at which maximum oxidative work can be done. Higher levels of lactate at rest would indicate increased anaerobic glycolysis at rest, which is consistent with capillary rarefaction.

Primary biliary cirrhosis is associated with Raynaud's phenomena, pruritus, sicca syndrome, osteoporosis, portal hypertension, neuropathy, and pancreatic insufficiency, and liver abnormalities are associated with rheumatic diseases. Elevated liver enzymes are a symptom of liver inflammation, and elevated liver enzymes are observed as an early symptom of "asymptomatic" primary biliary cirrhosis. Accordingly, the bacteria described herein may be used to treat liver inflammation.

Torre et al have reported that Alzheimer's disease (AD) is a microvascular disorder with neurological degeneration secondary to hypoperfusion, resulting in part from insufficient nitric oxide. (Review: Evidence that Alzheimer's disease is a microvascular disorder: the role of constitutive nitric oxide, Brain Research Reviews 34 (2000) 119-136.) Accordingly, the bacteria described herein may be used to treat AD.

Adverse health effects that are associated with hypertension may also be consequences of low basal NO. The decreased response to vasodilatation is also consistent with low basal NO. NO is a diffusible molecule that diffuses from a source to a sensor site where it has the signaling effect. With low NO levels, every NO source must produce more NO to generate an equivalent NO signal of a certain intensity a certain distance away. NO diffuses in three dimensions and the whole volume within that diffusion range must be raised to the level that will give the proper signal at the sensor location. This may result in higher NO levels at the source and between the source and the sensor. Adverse local effects of elevated NO near a source may then arise from too low a NO background. There is some evidence that this scenario actual occurs. In rat pancreatic islets, Henningsson et al have reported that inhibition of NOS with L-NAME increases total NO production through the induction of iNOS. (Chronic blockade of NO synthase paradoxically increases islet NO production and modulates islet hormone release. Am J Physiol Endocrinol Metab 279: E95-E107, 2000.) Increasing NO by increasing NOS activity will only work up to some limit. When NOS is activated but is not supplied with sufficient tetrahydrobiopterin (BH4) or L-arginine, it becomes "uncoupled" and generates superoxide (O2-) instead of NO. This O₂⁻ may then destroy NO. Attempting to produce NO at a rate that exceeds the supply of BH4 or L-arginine may instead decrease NO levels. This may result in positive feedback where low NO levels are made worse by stimulation of NOS, and uncoupled NOS generates significant O₂⁻ which causes local reactive oxygen species (ROS) damage such as is observed in atherosclerosis, end stage renal disease, Alzheimer's, and diabetes.

The bacteria described herein may also be used to delay the signs of aging. Caloric restriction extends lifespan, and Holloszy reported that restricting food intake to 70% of ad lib controls, prolongs life in sedentary rats from 858 to 1,051 days, almost 25%. (Mortality rate and longevity of food restricted exercising male rats: a reevaluation. J. Appl. Physiol. 82(2): 399-403, 1997.) The link between calorie restriction and prolonged life is well established, however, the causal mechanism is not. Lopez-Torres et al. reported that the examination of liver mitochondrial enzymes in rats indicates a reduction in H₂O₂ production due to reduced complex I activity associated with calorie restriction. (Influence Of Aging And Long-Term Caloric Restriction On Oxygen Radical Generation And Oxidative DNA Damage In Rat Liver Mitochondria. Free Radical Biology & Medicine Vol. 32 No 9 pp882-8899, 2002.) H₂O₂ is produced by dismutation of O₂⁻, which is a major ROS produced by the mitochondria during respiration. The main source of O₂⁻ has been suggested by Kushareva et al. and others to be complex I which catalyzes the NAD/NADH redox couple by reverse flow of electrons from complex III, the site of succinate reduction. The free radical theory, proposed by Beckman, of aging postulates, that free radical damage to cellular DNA, antioxidant systems and DNA repair systems accumulates with age and when critical systems are damaged beyond repair, death ensues. (The Free Radical Theory of Aging Matures. Physiol. Rev. 78: 547- 581, 1998.)

As an additional mechanism, NO has been demonstrated by Vasa et al. to activate telomerase and to delay senescence of endothelial cells. (Nitric Oxide Activates Telomerase and Delays Endothelial Cell Senescence. Circ Res. 2000;87:540-542.) Low basal NO will increase basal metabolic rate by disinhibition of cytochrome oxidase. Increased basal metabolism will also increase cell turn-over and growth rate. Capillary rarefaction, by inducing chronic hypoxia may increase free radical damage and may also increase cell turn-over, and so accelerate aging by both mechanisms.

In some aspects, it is appreciated that autotrophic ammonia-oxidizing bacteria may produce protective aspects for allergies and autoimmune disorders. The best known autoimmune disease is perhaps Diabetes Type 1, which results from the destruction of the insulin producing cells in the pancreas by the immune system. Recurrent pregnancy loss is also associated with autoimmune disorders where the number of positive autoimmune antibodies correlated positively with numbers recurrent pregnancy losses. Systemic Sclerosis, Primary Biliary Cirrhosis, autoimmune hepatitis, and the various rheumatic disorders are other examples of autoimmune disorders. Application of AOB was observed to reduce an allergy, hay fever, as described in WO/2005/030147.

One mechanism by which AOB may exert their protective effect on allergies and autoimmune disorders is through the production of nitric oxide, primarily through the regulatory inhibition of NF-KB and the prevention of activation of immune cells and the induction of inflammatory reactions. NF-KB is a transcription factor that up-regulates gene expression and many of these genes are associated with inflammation and the immune response including genes which cause the release of cytokines, chemokines, and various adhesion factors. These various immune factors cause the migration of immune cells to the site of their release resulting in the inflammation response. Constitutive NO production has been shown to inhibit NF-κB by stabilizing IκBα (an inhibitor of NF-κB) by preventing IκBα degradation.

Administration of an NO donor has been shown by Xu et al. to prevent the development of experimental allergic encephalomyelitis in rats. (SIN-1, a Nitric Oxide Donor, Ameliorates Experimental Allergic Encephalomyelitis in Lewis Rats in the Incipient Phase: The Importance of the Time Window. The Journal of Immunology, 2001, 166: 5810-5816.) In this study, it was demonstrated that administering an NO donor, reduced the infiltration of macrophages into the central nervous system, reduced the proliferation of blood mononuclear cells, and increased apoptosis of blood mononuclear cells. All of these results are expected to reduce the extent and severity of the induced autoimmune response.

Low basal NO may lead to autism via the mechanism that new connections in the brain are insufficiently formed as a result of insufficient basal nitric oxide. While not wishing to be bound in theory, formation of neural connections is modulated by NO. In these cases, any condition that lowers the range of NO diffusion may decrease the volume size of brain elements that can undergo connections. A brain which developed under conditions of low basal NO levels may be arranged in smaller volume elements because the reduced effective range of NO.

Additional symptoms exhibited in autistic individuals may also point to low NO as a cause, including increased pitch discrimination, gut disturbances, immune system dysfunction, reduced cerebral blood flow, increased glucose consumption of the brain, increased plasma lactate, attachment disorders, and humming. Each of these symptoms may be attributed to a low basal NO level.

Takashi Ohnishi et al. have reported that autistic individuals show decreased blood flow. Takashi Ohnishi et al., Abnormal regional cerebral blood flow in childhood autism. Brain (2000), 123, 1838-1844. J.M. Rumsey et al. have reported that autistic individuals have increased glucose consumption. Rumsey JM, Duara R, Grady C, Rapoport JL, Margolin RA, Rapoport SI, Cutler NR. Brain metabolism in autism. Resting cerebral glucose utilization rates as measured with positron emission tomography. Arch Gen Psychiatry, 1985 May;42(5):448-55 (abstract). D.C. Chugani has reported that autistic individuals have an increased plasma lactate levels. Chugani DC, et al., Evidence of altered energy metabolism in autistic children. Prog Neuropsychopharmacol Biol Psychiatry. 1999 May;23(4):635-41. The occurrence of these effects may be a result of capillary rarefaction in the brain, which may reduce blood flow and O₂ supply, such that some of the metabolic load of the brain may be produced through glycolysis instead of oxidative phosphorylation.

Nitric oxide has been demonstrated by B. A. Klyachko et al. to increase the excitability of neurons by increasing the after hyperpolarization through cGMP modification of ion channels. Vitaly A. Klyachko et al., cGMP-mediated facilitation in nerve terminals by enhancement of the spike after hyperpolarization. Neuron, Vol. 31, 1015-1025, September 27, 2001. C. Sandie et al. have shown that inhibition of NOS reduces startle. Carmen Sandi et al., Decreased spontaneous motor activity and startle response in nitric oxide synthase inhibitor-treated rats. European Journal of Pharmacology 277 (1995) 89-97. Attention-Deficit Hyperactivity Disorder (ADHD) has been modeled using the spontaneously hypertensive rat (SHR) and the Naples high-excitability (NHE) rat. Both of these models have been shown by Raffaele Aspide et al, to show increased attention deficits during periods of acute NOS inhibition. Raffaele Aspide et al., Non-selective attention and nitric oxide in putative animal models of attention-deficit hyperactivity disorder. Behavioral Brain Research 95 (1998) 123-133. Accordingly, the bacteria herein may be used in the treatment of ADHD.

Inhibition of NOS has also been shown by M. R. Dzoljic to inhibit sleep. M. R. Dzoljic, R. de Vries, R. van Leeuwen. Sleep and nitric oxide: effects of 7-nitro indazole, inhibitor of brain nitric oxide synthase. Brain Research 718 (1996) 145-150. G. Zoccoli has reported that a number of the physiological effects seen during sleep are altered when NOS is inhibited, including rapid eye movement and sleep-wake differences in cerebral circulation. G. Zoccoli, et al., Nitric oxide inhibition abolishes sleep-wake differences in cerebral circulation. Am. J. Physiol. Heart Circ Physiol 280: H2598-2606, 2001. NO donors have been shown by L. Kapas et al. to promote non-REM sleep, however, these increases persisted much longer than the persistence of the NO donor, suggesting perhaps a rebound effect.. Levente Kapas et al.. Nitric oxide donors SIN-1 and SNAP promote nonrapid-eye-movement sleep in rats. Brain Research Bullitin, vol 41, No 5, pp. 293-298, 1996. M. Rosaria et al., Central NO facilitates both penile erection and yawning. Maria Rosaria Melis and Antonio Argiolas. Role of central nitric oxide in the control of penile erection and yawning. Prog Neuro-Psychopharmacol & Biol. Phychiat. 1997, vol 21, pp 899-922. P. Tani et al, have reported that insomnia is a frequent finding in adults with Asperger's. Pekka Tani et al., Insomnia is a frequent finding in adults with Asperger's syndrome. BMC Psychiatry 2003, 3:12. Y. Hoshino has also observed sleep disturbances in autistic children. Hoshino Y, Watanabe H, Yashima Y, Kaneko M, Kumashiro H. An investigation on sleep disturbance of autistic children. Folia Psychiatr Neurol Jpn. 1984;38(1):45-51. (abstract) K.A. Schreck et al. has observed that the severity of sleep disturbances correlates with severity of autistic symptoms. Schreck KA, et al., Sleep problems as possible predictors of intensified symptoms of autism. Res Dev Disabil. 2004 Jan-Feb;25(1):57-66. (abstract). Accordingly, the bacteria herein may be used in the treatment of insomnia.

W. D. Ratnasooriya et al reported that inhibition of NOS in male rats reduces pre-coital activity, reduces libido, and reduces fertility. W. D. Ratnasooriya et al., Reduction in libido and fertility of male rats by administration of the nitric oxide (NO) synthase inhibitor N-nitro-L-arginine methyl ester. International journal of andrology, 23: 187-191 (2000).

It may be that a number of seemingly disparate disorders, characterized by ATP depletion and eventual organ failure are actually "caused" by nitropenia, caused by a global deficiency in basal nitric oxide. When this occurs in the heart, the result is dilative cardiomyopathy. When this occurs in the brain, the result is white matter hyperintensity, Alzheimer's, vascular depression, vascular dementia, Parkinson's, and the Lewy body dementias. When this occurs in the kidney, the result is end stage renal disease, when this occurs in the liver, the result is primary biliary cirrhosis. When this occurs in muscle, the consequence is fibromyaligia, Gulf War Syndrome, or chronic fatigue syndrome. When this occurs in the bowel, the consequence is ischemic bowel disease. When this occurs in the pancreas, the consequence is first type 2 diabetes, followed by chronic inflammation of the pancreas, followed by autoimmune attack of the pancreas (or pancreatic cancer), followed by type 1 diabetes. When this occurs in the connective tissue, the consequence is systemic sclerosis.

In the remnant kidney model of end stage renal disease, part of the kidney is removed, (either surgically or with a toxin) which increases the metabolic load on the remainder. Superoxide is generated to decrease NO and increase O₂ diffusion to the kidney mitochondria. Chronic overload results in progressive kidney capillary rarefaction and progressive kidney failure. In acute kidney failure, putting people in dialysis can give the kidney a "rest", and allows it to recover. In acute renal failure induced by rhabdomyolysis (muscle damage which releases myoglobin into the blood stream) kidney damage is characterized by ischemic damage. Myoglobin scavenges NO, just as hemoglobin does, and would cause vasoconstriction in the kidney leading to ischemia. Myoglobin would also induce local nitropenia and the cascade of events leading to further ATP depletion.

In some aspects, low NO levels lead to reduced mitochondrial biogenesis. Producing the same ATP at a reduced mitochondria density will result in an increase in O₂ consumption, or an accelerated basal metabolic rate. An accelerated basal metabolic rate is observed in a number of conditions, including: Sickle cell anemia, Congestive heart failure, Diabetes, Liver Cirrhosis, Crohn's disease, Amyotrophic lateral sclerosis, Obesity, End stage renal disease, Alzheimer's, and chronic obstructive pulmonary disease.

While some increased O₂ consumption might be productively used, in many of these conditions uncoupling protein is also up-regulated, indicating that at least part of the increased metabolic rate is due to inefficiency. Conditions where uncoupling protein is known to be up-regulated include obesity and diabetes.

With fewer mitochondria consuming O₂ to a lower O₂ concentration, the O₂ gradient driving O₂ diffusion is greater, so the O₂ diffusion path length can increase resulting in capillary rarefaction, which is observed in dilative cardiomyopathy, hypertension, diabetes type 2, and renal hypertension.

Copper, either as Cu2+ or as ceruloplasmin (CP) (the main Cu containing serum protein which is present at 0.38 g/L in adult sera and which is 0.32% Cu and contains 94% of the serum copper) catalyzes the formation of S-NO-thiols from NO and thiol containing groups (RSH). The Cu content of plasma is variable and is increased under conditions of infection. Berger et al. reported that the Cu and Zn content of burn-wound exudates is considerable with patients with 1/3 of their skin burned, losing 20 to 40% of normal body Cu and 5 to 10% of Zn content in 7 days. (Cutaneous copper and zinc losses in burns. Burns. 1992 Oct;18(5):373-80.) If the patients skin were colonized by AOB, wound exudates which contains urea and Fe, Cu, and Zn that AOB need, would be converted into NO and nitrite, greatly supplementing the local production of NO by iNOS, without consuming resources (such as O₂ and L-arginine) in the metabolically challenged wound. A high production of NO and nitrite by AOB on the surface of a wound would be expected to inhibit infection, especially by anaerobic bacteria such as the Clostridia which cause tetanus, gas gangrene, and botulism.

The practice of the present disclosure may employ, unless otherwise indicated, conventional methods of immunology, molecular biology, and recombinant DNA techniques within the skill of the art. Such techniques are explained fully in the literature. See, e.g., Sambrook, et al. Molecular Cloning: A Laboratory Manual (Current Edition); and Current Protocols in Molecular Biology (F.M. Ausubel, et al. eds., current edition).

While specific instances of the subject disclosure have been discussed, the above specification is illustrative and not restrictive. Many variations of the disclosure will become apparent to those skilled in the art upon review of this specification and the claims below. The full scope of the disclosure should be determined by reference to the claims, along with their full scope of equivalents, and the specification, along with such variations.

## Claims

1. A method of evaluating a preparation for the level of ammonia oxidizing bacteria, comprising:
(a) measuring an amount or a concentration of ammonia oxidizing bacteria in the preparation to provide a value;
(b) comparing the value to a range of values corresponding to a pre-determined range of amounts or concentrations of ammonia oxidizing bacteria; and
(c) determining if the value is a value in the range of values corresponding to the pre-determined range of amounts or concentrations of ammonia oxidizing bacteria, wherein:
(i) if the value is in the range of values corresponding to the pre-determined range of amounts or concentrations of ammonia oxidizing bacteria, classifying the product as accepted; or
(ii) if the value is outside the range of values corresponding to the pre-determined range of amounts or concentrations of ammonia oxidizing bacteria, classifying the product as not accepted;
wherein the preparation is a cosmetic product, a therapeutic product or a consumer product, or is in the process of being prepared or manufactured as a cosmetic product, a therapeutic product or a consumer product.

2. The method of claim 1, further comprising classifying the preparation as requiring addition of ammonia oxidizing bacteria or not requiring addition of ammonia oxidizing bacteria.

3. The method of claims 1-2, wherein if the value is outside the range of values corresponding to the pre-determined range of amounts or concentrations of ammonia oxidizing bacteria, adding at least one of ammonia oxidizing bacteria, ammonia, ammonium salts, and urea.

4. The method of any one of claims 1-3, further comprising evaluating the product for contaminating organisms and/or pathogenic organisms, optionally wherein the pathogenic organisms are selected from the group consisting of *Psuedomonas aeruginosa, Staphylococcus aureus, Streptococcus pyogenes, Acinetobacter baumannii, Propionibacteria,* and *Stenotrophomonas,* and combinations thereof.

5. The method of any one of claims 1-4, further comprising culturing the ammonia oxidizing bacteria.

6. The method of any one of claims 1-5, further comprising selecting a sample from the product and conducting testing on the sample.

7. The method of claim 6, wherein said testing comprises:
(i) measuring the pH of the preparation;
(ii) measuring the rate of oxidation of ammonia, ammonium ions and/or urea;
(iii) measuring a level of nitrite;
(iv) measuring an amount or concentration of a protein;
(v) measuring the optical density (OD);
(vi) measuring the level of waste products; and/or
(vii) measuring the level of nutrients.

8. The method of any one of claims 1-4, wherein the range of values corresponding to the pre-determined range of amounts or concentrations of ammonia oxidizing bacteria is:
(i) between about 10⁸ CFU/L to about 10¹⁴ CFU/L;
(ii) between about 50 milligrams (mg) and about 1000 mg of ammonia oxidizing bacteria; and/or
(iii) a therapeutically effective amount of ammonia oxidizing bacteria.

9. The method of any one of claims 1-8, wherein the preparation is for use as:
(i) a treatment or prevention of a skin disorder,
(ii) a treatment or prevention of a disease or condition associated with low nitrite levels,
(iii) a treatment or prevention of body odor,
(iv) a treatment to supply nitric oxide to a subject, or
(v) a treatment to inhibit microbial growth.

10. The method of any one of claims 1-9, wherein the ammonia oxidizing bacteria is selected from the group consisting of *Nitrosomonas, Nitrosococcus, Nitrosospira, Nitrosocystis, Nitrosolobus, Nitrosovibrio,* and combinations thereof.

11. The method of any one of claims 1-10, further comprising evaluating the preparation from a first batch, *e.g.,* a first batch for commercial release, and optionally further comprising evaluating the preparation from a second batch, *e.g.,* a second batch for commercial release.

12. The method of any one of claims 1-11, further comprising evaluating the preparation from a plurality of batches to determine if each batch within the plurality meets the predetermined range of amounts or concentrations of ammonia oxidizing bacteria, optionally further comprising releasing the plurality of batches, optionally wherein the plurality of batches was prepared simultaneously.

13. The method of claim 12, wherein the plurality of batches are evaluated at pre-determined times or batch intervals, to evaluate the consistency of preparations over time.

14. The method of any one of claims 1-13, wherein the preparation is for use in the treatment of at least one of HIV, dermatitis, infection in an ulcer, *e.g.,* venous ulcer, *e.g.,* leg ulcer, *e.g.,* venous leg ulcer, *e.g.* infection in a diabetic foot ulcer, atopic dermatitis, acne, *e.g.,* acne vulgaris, eczema, contact dermatitis, allergic reaction, psoriasis, uticaria, rosacea, skin infections, vascular disease, vaginal yeast infection, a sexually transmitted disease, heart disease, atherosclerosis, baldness, leg ulcers secondary to diabetes or confinement to bed, angina, particularly chronic, stable angina pectoris, ischemic diseases, congestive heart failure, myocardial infarction, ischemia reperfusion injury, laminitis, hypertension, hypertrophic organ degeneration, Raynaud's phenomenon, fibrosis, fibrotic organ degeneration, allergies, autoimmune sensitization, end stage renal disease, obesity, impotence, pneumonia, primary immunodeficiency, epidermal lysis bulosa or cancer.

15. The method of claim 14, wherein the preparation is for use in the treatment of at least one of an infection in an ulcer, *e.g.,* venous ulcer, *e.g.,* leg ulcer, *e.g.,* venous leg ulcer, *e.g.* infection in a diabetic foot ulcer, atopic dermatitis, acne, *e.g.,* acne vulgaris, eczema, psoriasis, uticaria, rosacea, and skin infections.

16. The method of any one of claims 1-15, wherein the preparation comprises, is provided as, or is disposed in at least one of a baby product, *e.g*., a baby shampoo, a baby lotion, a baby oil, a baby powder, a baby cream; a bath preparation, *e.g*., a bath oil, a tablet, a salt, a bubble bath, a bath capsule; an eye makeup preparation, *e.g*., an eyebrow pencil, an eyeliner, an eye shadow, an eye lotion, an eye makeup remover, a mascara; a fragrance preparation, *e.g*., a colognes, a toilet water, a perfume, a powder (dusting and talcum), a sachet; hair preparations, *e.g*., hair conditioners, hair sprays, hair straighteners, permanent waves, rinses, shampoos, tonics, dressings, hair grooming aids, wave sets; hair coloring preparations, *e.g*., hair dyes and colors, hair tints, coloring hair rinses, coloring hair shampoos, hair lighteners with color, hair bleaches; makeup preparations, *e.g*., face powders, foundations, leg and body paints, lipstick, makeup bases, rouges, makeup fixatives; manicuring preparations, *e.g*., basecoats and undercoats, cuticle softeners, nail creams and lotions, nail extenders, nail polish and enamel, nail polish and enamel removers; oral hygiene products, *e.g*., dentrifices, mouthwashes and breath fresheners; bath soaps and detergents, deodorants, douches, feminine hygiene deodorants; shaving preparations, *e.g*., aftershave lotions, beard softeners, talcum, preshave lotions, shaving cream, shaving soap; skin care preparations, *e.g*., cleansing, depilatories, face and neck, body and hand, foot powders and sprays, moisturizing, night preparations, paste masks, skin fresheners; and suntan preparations, *e.g*., gels, creams, and liquids, and indoor tanning preparations.

## Patentansprüche

1. Ein Verfahren zur Auswertung einer Zubereitung auf ein Niveau von ammoniakoxidierenden Bakterien, das Folgendes beinhaltet:
(a) Messen einer Menge oder einer Konzentration von ammoniakoxidierenden Bakterien in der Zubereitung, um einen Wert bereitzustellen;
(b) Vergleichen des Werts mit einem Wertebereich, der einem vorbestimmten Bereich von Mengen oder Konzentrationen von ammoniakoxidierenden Bakterien entspricht; und
(c) Bestimmen, ob der Wert ein Wert in dem Wertebereich ist, der dem vorbestimmten Bereich von Mengen oder Konzentrationen von ammoniakoxidierenden Bakterien entspricht, wobei:
(i) wenn der Wert in dem Wertebereich liegt, der dem vorbestimmten Bereich von Mengen oder Konzentrationen von ammoniakoxidierenden Bakterien entspricht, Klassifizieren des Produkts als akzeptiert; oder
(ii) wenn der Wert außerhalb des Wertebereichs liegt, der dem vorbestimmten Bereich von Mengen oder Konzentrationen von ammoniakoxidierenden Bakterien entspricht, Klassifizieren des Produkts als nicht akzeptiert;
wobei die Zubereitung ein kosmetisches Produkt, ein therapeutisches Produkt oder ein Verbrauchsprodukt ist oder im Begriff ist, als ein kosmetisches Produkt, ein therapeutisches Produkt oder ein Verbrauchsprodukt zubereitet oder hergestellt zu werden.

2. Verfahren nach Anspruch 1, das ferner das Klassifizieren der Zubereitung als die Zugabe von ammoniakoxidierenden Bakterien erforderlich machend oder die Zugabe von ammoniakoxidierenden Bakterien nicht erforderlich machend beinhaltet.

3. Verfahren nach Ansprüchen 1-2, wobei, wenn der Wert außerhalb des Wertebereichs liegt, der dem vorbestimmten Bereich von Mengen oder Konzentrationen von ammoniakoxidierenden Bakterien entspricht, Zugeben von mindestens einem von ammoniakoxidierenden Bakterien, Ammoniak, Ammoniumsalzen und Harnstoff.

4. Verfahren nach einem der Ansprüche 1-3, das ferner das Auswerten des Produkts in Bezug auf verunreinigende Organismen und/oder Erregerorganismen beinhaltet, wobei die Erregerorganismen optional aus der Gruppe ausgewählt sind, die aus *Pseudomonas aeruginosa, Staphylococcus aureus, Streptococcus pyogenes, Acinetobacter baumannii, Propionibakterien* und *Stenotrophomonas* und Kombinationen davon besteht.

5. Verfahren nach einem der Ansprüche 1-4, das ferner das Kultivieren der ammoniakoxidierenden Bakterien beinhaltet.

6. Verfahren nach einem der Ansprüche 1-5, das ferner das Auswählen einer Probe von dem Produkt und das Durchführen von Tests an der Probe beinhaltet.

7. Verfahren nach Anspruch 6, wobei das Testen Folgendes beinhaltet:
(i) Messen des pH-Werts der Zubereitung;
(ii) Messen der Rate der Oxidation von Ammoniak, Ammoniumionen und/oder Harnstoff;
(iii) Messen eines Niveaus von Nitrit;
(iv) Messen einer Menge oder Konzentration eines Proteins;
(v) Messen der optischen Dichte (OD);
(vi) Messen des Niveaus von Abfallprodukten; und/oder
(vii) Messen des Niveaus von Nährstoffen.

8. Verfahren nach einem der Ansprüche 1-4, wobei der Wertebereich, der dem vorbestimmten Bereich von Mengen oder Konzentrationen von ammoniakoxidierenden Bakterien entspricht, wie folgt ist:
(i) zwischen etwa 10⁸ CFU/l bis etwa 10¹⁴ CFU/l;
(ii) zwischen etwa 50 Milligramm (mg) und etwa 1000 mg von ammoniakoxidierenden Bakterien; und/oder
(iii) einer therapeutisch wirksamen Menge von ammoniakoxidierenden Bakterien.

9. Verfahren nach einem der Ansprüche 1-8, wobei die Zubereitung zur Verwendung als Folgendes dient:
(i) Behandlung oder Prävention einer Hauterkrankung,
(ii) Behandlung oder Prävention einer Erkrankung oder eines Krankheitszustands in Verbindung mit niedrigen Nitritspiegeln,
(iii) Behandlung oder Prävention von Körpergeruch,
(iv) Behandlung zum Zuführen von Stickstoffoxid zu einem Individuum oder
(v) Behandlung zum Hemmen des Wachstums von Mikroben.

10. Verfahren nach einem der Ansprüche 1-9, wobei die ammoniakoxidierenden Bakterien aus der Gruppe ausgewählt sind, die aus *Nitrosomonas, Nitrosococcus, Nitrosospira, Nitrosocystis, Nitrosolobus, Nitrosovibrio* und Kombinationen davon besteht.

11. Verfahren nach einem der Ansprüche 1-10, das ferner das Auswerten der Zubereitung von einer ersten Charge, z. B. einer ersten Charge für die kommerzielle Freigabe, beinhaltet und optional ferner das Auswerten der Zubereitung aus einer zweiten Charge, z. B. einer zweiten Charge für die kommerzielle Freigabe, beinhaltet.

12. Verfahren nach einem der Ansprüche 1-11, das ferner das Auswerten der Zubereitung aus einer Vielzahl von Chargen beinhaltet, um zu bestimmen, ob jede Charge innerhalb der Vielzahl in den vorbestimmten Bereich von Mengen oder Konzentrationen von ammoniakoxidierenden Bakterien fällt, das ferner optional das Freigeben der Vielzahl von Chargen beinhaltet, wobei die Zubereitung der Vielzahl von Chargen optional gleichzeitig erfolgte.

13. Verfahren nach Anspruch 12, wobei die Vielzahl von Chargen zu vorbestimmten Zeiten oder Chargenintervallen ausgewertet wird, um auszuwerten, ob die Zubereitungen im Verlauf der Zeit konsistent sind.

14. Verfahren nach einem der Ansprüche 1-13, wobei die Zubereitung zur Verwendung bei der Behandlung von mindestens einem von Folgenden dient: HIV, Dermatitis, Infektion in einem Geschwür, z. B. einem venösen Geschwür, z. B. Beingeschwür, z. B. einem venösen Beingeschwür, z. B. Infektion in einem diabetischen Fußgeschwür, atopischer Dermatitis, Akne, z. B. Acne vulgaris, Ekzem, Kontaktekzem, allergischer Reaktion, Psoriasis, Urtikaria, Rosacea, Hautinfektionen, Gefäßerkrankung, vaginaler Hefepilzinfektion, einer sexuell übertragenen Erkrankung, Herzerkrankung, Atherosklerose, Kahlköpfigkeit, Beingeschwüren infolge Diabetes oder Bettlägerigkeit, Angina, insbesondere chronischer, stabiler Angina pectoris, ischämischen Erkrankungen, Herzinsuffizienz, Herzinfarkt, ischämischer Reperfusionsschädigung, Laminitis, Hypertonie, hypertropher Organdegeneration, Raynaud-Phänomen, Fibrose, fibrotischer Organdegeneration, Allergien, Autoimmunsensibilisierung, terminaler Niereninsuffizienz, Fettleibigkeit, Impotenz, Pneumonie, primärem Immundefekt, Epidermolysis bullosa oder Krebs.

15. Verfahren nach Anspruch 14, wobei die Zubereitung zur Verwendung bei der Behandlung von mindestens einem von den Folgenden dient: einer Infektion in einem Geschwür, z. B. einem venösen Geschwür, z. B. Beingeschwür, z. B. einem venösen Beingeschwür, z. B. Infektion in einem diabetischen Fußgeschwür, atopischer Dermatitis, Akne, z. B. Acne vulgaris, Ekzem, Psoriasis, Urtikaria, Rosacea, Hautinfektionen.

16. Verfahren nach einem der Ansprüche 1-15, wobei die Zubereitung mindestens eines der Folgenden beinhaltet, als mindestens eines der Folgenden bereitgestellt wird oder in mindestens einem der Folgenden angeordnet ist: ein Babyprodukt, z. B. ein Babyshampoo, eine Babylotion, ein Babyöl, ein Babypuder, eine Babycreme; eine Badezubereitung, z. B. ein Badeöl, eine Badetablette, ein Badesalz, ein Schaumbad, eine Badekapsel; eine Augen-Makeup-Zubereitung, z. B. ein Augenbrauenstift, ein Lidstrich, ein Lidschatten, eine Augenlotion, ein Augen-Makeup-Entferner, eine Wimperntusche; eine Duftzubereitung, z. B. ein Eau de Cologne, ein Eau de Toilette, ein Parfum, ein Puder (Körperpuder und Talkumpuder), ein Duftkissen; Haarzubereitungen, z. B. Haarbalsam, Haarsprays, Haarglättmittel, Dauerwellen, Haarspülungen, Shampoos, Haarkuren, Haarpflegemittel, Haarkämmhilfen, Wellmittel; Haarfärbezubereitungen, z. B. Haarfärbemittel und Haarkoloration, Haartönungen, Haarfarbspülungen, Haarfärbeshampoos, Haaraufheller mit Farbe, Haarbleichmittel; Makeup-Zubereitungen, z. B. Gesichtspuder, Grundierung, Bein- und Körperfärbemittel, Lippenstift, Schminkgrundlagen, Rouges, Makeup-Fixiermittel; Manikürezubereitungen, z. B. Base-Coats und Grundierungen, Nagelhauterweichungsmittel, Nagelcremes und Lotionen, Nagelverlängerungen, Nagellack und Enamel, Nagellack- und Enamelentferner; Mundhygieneprodukte, z. B. Zahnreinigungsmittel, Mundspülungen und Mundwasser; Badeseifen und -reinigungsmittel, Deodorants, Intimduschen, Intimpflegedeodorants; Rasierzubereitungen, z. B. Aftershavelotionen, Barterweichungsmittel, Talkum, Preshave-Lotionen, Rasiercreme, Rasierseife; Hautpflegezubereitungen, z. B. Reinigungspräparate, Enthaarungsmittel, Gesichts- und Hals-, Körper- und Hand-, Fußpuder und -sprays, Feuchtigkeitsmittel, Nachtzubereitungen, Mitessermasken, Hauterfrischungsmittel; und Sonnenschutzzubereitungen, z. B.
Sonnenschutzgele, -cremes und -milch, und Bräunungszubereitungen.

## Revendications

1. Procédé d'évaluation d'une préparation pour déterminer le niveau de bactéries oxydant l'ammoniac, comprenant :
(a) la mesure d'une quantité ou d'une concentration de bactéries oxydant l'ammoniac dans la préparation pour produire une valeur ;
(b) une comparaison de la valeur à une gamme de valeurs correspondant à une gamme prédéterminée de quantités ou de concentrations de bactéries oxydant l'ammoniac ; et
(c) le fait de déterminer si la valeur est une valeur dans la gamme de valeurs correspondant à la gamme prédéterminée de quantités ou de concentrations de bactéries oxydant l'ammoniac, dans lequel :
(i) si la valeur se situe dans la gamme de valeurs correspondant à la gamme prédéterminée de quantités ou de concentrations de bactéries oxydant l'ammoniac, le classement du produit comme accepté ; ou
(ii) si la valeur est en dehors de la gamme de valeurs correspondant à la gamme prédéterminée de quantités ou de concentrations de bactéries oxydant l'ammoniac, le classement du produit comme non accepté ;
dans lequel la préparation est un produit cosmétique, un produit thérapeutique ou un produit de consommation, ou est en cours de préparation ou de fabrication comme produit cosmétique, produit thérapeutique ou produit de consommation.

2. Procédé selon la revendication 1, comprenant en outre un classement de la préparation comme nécessitant une addition de bactéries oxydant l'ammoniac ou ne nécessitant pas d'addition de bactéries oxydant l'ammoniac.

3. Procédé selon les revendications 1 ou 2, dans lequel si la valeur est en dehors de la gamme de valeurs correspondant à la gamme prédéterminée de quantités ou de concentrations de bactéries oxydant l'ammoniac, on ajoute au moins l'un de bactéries oxydant l'ammoniac, de l'ammoniac, de sels d'ammonium et de l'urée.

4. Procédé selon l'une quelconque des revendications 1 à 3, comprenant en outre une évaluation du produit pour déterminer la présence d'organismes contaminants et/ou d'organismes pathogènes, optionnellement dans lequel les organismes pathogènes sont sélectionnés dans le groupe constitué de *Pseudomonas aeruginosa, Staphylococcus aureus, Streptococcus pyogenes, Acinetobacter baumannii, Propionibacteria,* et *Stenotrophomonas,* et de combinaisons de ceux-ci.

5. Procédé selon l'une quelconque des revendications 1 à 4, comprenant en outre une mise en culture des bactéries oxydant l'ammoniac.

6. Procédé selon l'une quelconque des revendications 1 à 5, comprenant en outre la sélection d'un échantillon à partir du produit et la réalisation de tests sur l'échantillon.

7. Procédé selon la revendication 6, dans lequel lesdits tests comprennent :
(i) une mesure du pH de la préparation ;
(ii) une mesure du taux d'oxydation de l'ammoniac, des ions ammonium et/ou de l'urée ;
(iii) la mesure d'un niveau de nitrite ;
(iv) la mesure d'une quantité ou d'une concentration d'une protéine ;
(v) une mesure de la densité optique (DO) ;
(vi) une mesure du niveau de déchets ; et/ou
(vii) une mesure du niveau d'éléments nutritifs.

8. Procédé selon l'une quelconque des revendications 1 à 4, dans lequel la gamme de valeurs correspondant à la gamme prédéterminée de quantités ou de concentrations de bactéries oxydant l'ammoniac est :
(i) entre environ 10⁸ UFC/l et environ 10¹⁴ UFC/l ;
(ii) entre environ 50 milligrammes (mg) et environ 1 000 mg de bactéries oxydant l'ammoniac ; et/ou
(iii) une quantité thérapeutiquement efficace de bactéries oxydant l'ammoniac.

9. Procédé selon l'une quelconque des revendications 1 à 8, dans lequel la préparation est destinée à une utilisation comme :
(i) traitement ou prévention d'une affection cutanée,
(ii) traitement ou prévention d'une maladie ou d'une affection associée à des niveaux de nitrite bas,
(iii) traitement ou prévention d'odeurs corporelles,
(iv) traitement visant à apporter de l'oxyde nitrique à un sujet, ou
(v) traitement visant à inhiber la croissance microbienne.

10. Procédé selon l'une quelconque des revendications 1 à 9, dans lequel les bactéries oxydant l'ammoniac sont sélectionnées dans le groupe constitué de *Nitrosomonas, Nitrosococcus, Nitrosospira, Nitrosocystis, Nitrosolobus, Nitrosovibrio,* et de combinaisons de ceux-ci.

11. Procédé selon l'une quelconque des revendications 1 à 10, comprenant en outre une évaluation de la préparation à partir d'un premier lot, par exemple un premier lot pour libération commerciale, et comprenant optionnellement en outre une évaluation de la préparation à partir d'un deuxième lot, par exemple un deuxième lot pour libération commerciale.

12. Procédé selon l'une quelconque des revendications 1 à 11, comprenant en outre une évaluation de la préparation à partir d'une pluralité de lots pour déterminer si chaque lot dans la pluralité se situe dans la gamme prédéterminée de quantités ou de concentrations de bactéries oxydant l'ammoniac, comprenant optionnellement en outre une libération de la pluralité de lots, optionnellement dans lequel les lots de la pluralité ont été préparés simultanément.

13. Procédé selon la revendication 12, dans lequel les lots de la pluralité sont évalués à des moments prédéterminés ou selon des intervalles prédéterminés entre les lots, pour évaluer l'uniformité des préparations dans le temps.

14. Procédé selon l'une quelconque des revendications 1 à 13, dans lequel la préparation est destinée à une utilisation dans le traitement d'au moins l'un du VIH, d'une dermatite, d'une infection dans un ulcère, par exemple un ulcère veineux, par exemple un ulcère de jambe, par exemple un ulcère veineux de jambe, par exemple une infection dans un ulcère diabétique du pied, d'une dermatite atopique, d'une acné, par exemple une acné juvénile, d'un eczéma, d'une dermatite de contact, d'une réaction allergique, d'un psoriasis, d'une urticaire, d'une rosacée, d'infections cutanées, de maladies vasculaires, d'infections vaginales à levures, d'une maladie sexuellement transmissible, de maladies cardiaques, d'une athérosclérose, d'une calvitie, d'ulcères de jambe secondaires à un diabète ou à un alitement, d'une angine, particulièrement chronique, d'un angor pectoris stable, de maladies ischémiques, d'une insuffisance cardiaque congestive, d'un infarctus du myocarde, d'une lésion de reperfusion ischémique, d'une fourbure, d'une hypertension, d'une dégénérescence organique hypertrophique, de la maladie de Raynaud, d'une fibrose, d'une dégénérescence organique fibrotique, d'allergies, d'une sensibilisation auto-immune, d'une maladie rénale au stade terminal, d'une obésité, d'une impuissance, d'une pneumonie, d'une immunodéficience primaire, d'une épidermolyse bulleuse ou d'un cancer.

15. Procédé selon la revendication 14, dans lequel la préparation est destinée à une utilisation dans le traitement d'au moins l'un d'une infection dans un ulcère, par exemple un ulcère veineux, par exemple un ulcère de jambe, par exemple un ulcère veineux de jambe, par exemple une infection dans un ulcère diabétique du pied, d'une dermatite atopique, d'une acné, par exemple une acné juvénile, d'un eczéma, d'un psoriasis, d'une urticaire, d'une rosacée, et d'infections cutanées.

16. Procédé selon l'une quelconque des revendications 1 à 15, dans lequel la préparation comprend, est fournie sous forme de, ou est disposée dans, au moins l'un d'un produit pour bébé, par exemple un shampooing pour bébé, une lotion pour bébé, une huile pour bébé, une poudre pour bébé, une crème pour bébé ; d'une préparation pour le bain, par exemple une huile de bain, une tablette, un sel, un bain moussant, une capsule de bain ; d'une préparation de maquillage pour les yeux, par exemple un crayon à sourcils, un eye-liner, une ombre à paupières, une lotion pour les yeux, un démaquillant pour les yeux, un mascara ; d'une préparation parfumée, par exemple une eau de Cologne, une eau de toilette, un parfum, une poudre (poudres pour le corps et talcs), un sachet ; de préparations capillaires, par exemple des conditionneurs pour les cheveux, des laques, des produits lissants pour les cheveux, des produits pour permanentes, des rinçages, des shampooings, des toniques, des masques, des produits de grooming pour les cheveux, des produits bouclants ; de préparations de coloration des cheveux, par exemple des teintures et des couleurs pour les cheveux, des produits apportant des reflets aux cheveux, des rinçages colorants pour les cheveux, des shampooings colorants, des éclaircissants colorant les cheveux, des produits décolorants pour les cheveux ; de préparations de maquillage, par exemple des poudres pour le visage, des fonds de teint, des peintures pour les jambes et le corps, des rouges à lèvres, des bases de maquillage, des blushes, des produits pour fixer le maquillage ; de préparations de manucure, par exemple des couches de base et des sous-couches, des produits pour ramollir les cuticules, des crèmes et des lotions pour les ongles, des faux-ongles, des vernis et laques à ongles, des dissolvants pour les vernis et laques à ongles ; de produits d'hygiène bucco-dentaire, par exemple des dentifrices, des rince-bouche et des rafraîchisseurs d'haleine ; de savons et de détergents pour le bain, de déodorants, de nettoyants intimes, de déodorants d'hygiène féminine ; de préparations de rasage, par exemple des lotions après-rasage, des adoucisseurs de barbe, des talcs, des lotions pré-rasage, des crèmes à raser, des savons à raser ; de préparations pour les soins de la peau, par exemple des produits nettoyants, des dépilatoires, des produits pour le visage et le cou, des produits pour le corps et les mains, des poudres et pulvérisations pour les pieds, des produits hydratants, des préparations à appliquer le soir, des masques sous forme de pâte, des produits rafraîchissants pour la peau ; et de préparations solaires, par exemple des gels, des crèmes et des liquides, et des préparations auto-bronzantes.
